Europäisches Patentamt

European Patent Office

Office européen des brevets

(11) Publication number: **0 310 056**
**A2**

# EUROPEAN PATENT APPLICATION

(21) Application number: 88116054.3

(22) Date of filing: 29.09.88

(51) Int. Cl.⁴: **C12P 21/02** , **C07K 15/06** ,
**//A61K35/26**

(30) Priority: 30.09.87 JP 248616/87

(43) Date of publication of application:
05.04.89 Bulletin 89/14

(84) Designated Contracting States:
AT BE CH DE ES FR GB GR IT LI LU NL SE

(71) Applicant: SHIONOGI SEIYAKU KABUSHIKI
KAISHA trading under the name of
SHIONOGI & CO. LTD.
12, 3-chome, Dosho-machi Higashi-ku
Osaka(JP)

Applicant: Fujiwara, Hiromi
1-19-12, Mikageyamate Higashinada-ku
Kobe-shi Hyogo(JP)

(72) Inventor: Hamaoka, Toshiyuki
4-12-16, Mayumi
Ikoma-shi Nara(JP)
Inventor: Itoh, Tsunetoshi
1-3-2-202, Mikamine
Sendai-shi Miyagi(JP)
Inventor: Fujiwara, Hiromi
1-19-12, Mikageyamate Higashinada-ku
Kobe-shi Hyogo(JP)
Inventor: Doi, Hideyuki
1-7-13, Kashiwagi
Sendai-shi Miyagi(JP)

(74) Representative: Vossius & Partner
Siebertstrasse 4 P.O. Box 86 07 67
D-8000 München 86(DE)

(54) Thymic stroma-derived T cell growth factor and process for its production.

(57) Disclosed is a novel T cell growth factor which is derived from a thymic stroma and promotes the growth of T cell clones and/or thymocytes and a process for its production which comprises culturing thymic stroma cells in a culture medium and collecting a factor promoting the growth of T cells form the supernatant of said culture medium.

# THYMIC STROMA-DERIVED T CELL GROWTH FACTOR AND PROCESS FOR ITS PRODUCTION

This invention relates to a thymic stroma-derived T cell growth factor (hereafter abbreviated as TSTGF) and to its production.

It has been established that the thymic stroma is an essential microenvironment for the proper development of precursors of the T cell lineage into immunocompetent peripheral T lymphocytes (Phisiol. Rev. 47:437, 1967, Cancer Res. 28:21, 1968, Ann. N.Y. Acad. Sci. 249:493, 1975, Immunol. Rev. 42:138, 1978, J. Immunol. 123:984, 1979). The thymic stroma is composed of at least three components: fibroblastic, epithelial and macrophage-like cells (J. Reticuloendothel. Soc. 26:385, 1979), all of which may be involved in the differentiation and/or proliferation of the T cell lineage in the thymus (J. Immunol. 124:1433, 1980, Nature 287:44, 1980, J. Immunol. 134:2155, 1985, Thymus 2:193, 1981). In fact, it has been reported that primary explants of thymic stromal tissue are capable of effecting the maturation of immature thymocytes to responding cells in mixed lymphocyte reaction and in the stimulation with phytohemagglutinin (J. Exp. Med. 136:1483. 1972, J. Immunol. 121:1861, 1978).

The thymic stroma may exert its effect on functionally immature thymocytes not only through processes involving cell contact, but also through events that are mediated by humoral factors. In fact, a variety of soluble mediators derived from thymic stromal components has been reported to affect the maturation of immature thymocytes (Thymus 1:163, 1979, Prog. Allergy 21:342, 1976, In Biological Responses in Cancer, Vol. I, E. Mihick, ed. Pleum. New York, P. 219, 1982), expression of marker antigen on a cell surface, and reactivities in proliferative responses. Especially, there is a report by Kruisbeek et al on this sort of factor present in the culture supernatant (SN) of primary explants of thymus itself. (J. Immunol. 125:995, 1980; Eur. J. Immunol. 7:375, 1977; J. Immunol. 123:984, 1979). These soluble factors, which are considered to be derived from thymic stroma, have also been obtained in the supernatant collected from cultured explants of thymic stromal tissue. However, the lack of pure cell line-cultures has complicated interpretation of the exact cellular origin and the precise capability of these factors. Thus, for the final elucidation of the functions of the component parts of thymic stroma, it is necessary to utilize various cloned T cell lines as targets of the factors and also to establish purely cultured cell lines from thymic stroma. These make it possible to perform subsequent experiments in detail. As a study along this line, Glimcher et al (J. Immunol. 17:1, 1983) and Beardsley (Proc. Natl. Acad. Sci. USA, 80:6005, 1983) have recently reported the production of factors capable of inducing T cell maturation to killer T cell by a thymic epithelial cell line or cloned T cell lines into which had been given the capability of proliferation with the use of SV40 and epidermal growth factor (EGF). In these reports, however, the exact physicochemical properties of the said factors have not yet been clarified. We have found that the thymic stroma-derived cell line (TSCF) recently established by us produces a factor having new properties capable of inducing proliferation of antigen-specific and interleukin (IL) 2- dependent T cell clones even in the absence of corresponding antigen and IL2. The factor with such new properties has not yet been reported.

Namely, it has been shown that the thymic stroma cell line in a fibroblastic form (TSCF) which was established by us produces a factor capable of inducing the proliferation of various T cell clones. In addition, it has become clear that the said factor is entirely different from interleukin (IL) 1, 2, 3, 4, 5 and 6, and also from known cytokines including interferon in terms of the function. Moreover, it has been proven that this factor is also produced by the primary explant of thymic stroma cell. Therefore, this invention provides a thymic stroma-derived T cell growth factor having new properties and a method for preparing it.

Various cytokines and monokines have so far been developed as T cell growth factors and are being applied to clinical use. However, there has not been attained so much effect as originally expected and discovery of other T cell growth factors is urgently needed at present. Under such circumstances, the presentation of a T cell growth factor having novel properties will make a new development in the clinical application of T cell growth factors and will contribute to the the medicines attempting the treatment of diseases of immunodeficiency, the artificial strengthening of the immune response in various infections and cancers as well as the accelerated repopulation of T lymphocytes after bone marrow reconstruction.

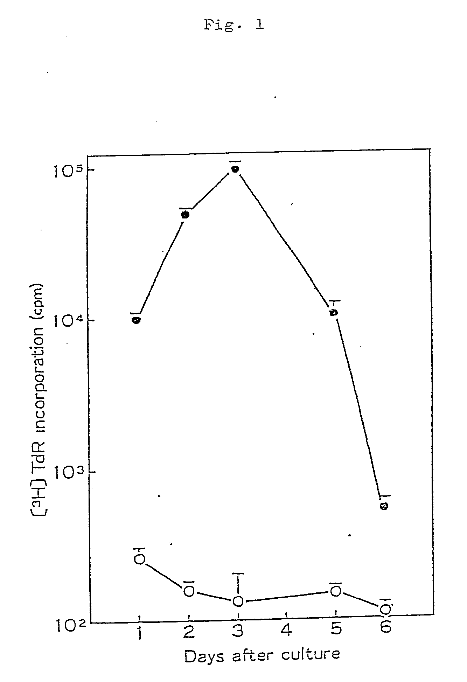

Fig. 1 shows proliferation of 9-16 Th clone by TSCF culture SN. 9-16 Th clone (1 x 10$^4$ cells/well) was cultured in 96-well flat-bottomed microplates in the presence ($\bullet$) or absence ($\circ$) of culture SN (25%). Various days after culturing, cells were pulsed with [$^3$H] thymidine (TdR) for 7 hr and then harvested. Radioactivity incorporated into cell DNA was expressed by the mean [$^3$H] uptake and S.E. of triplicate cultures/group.

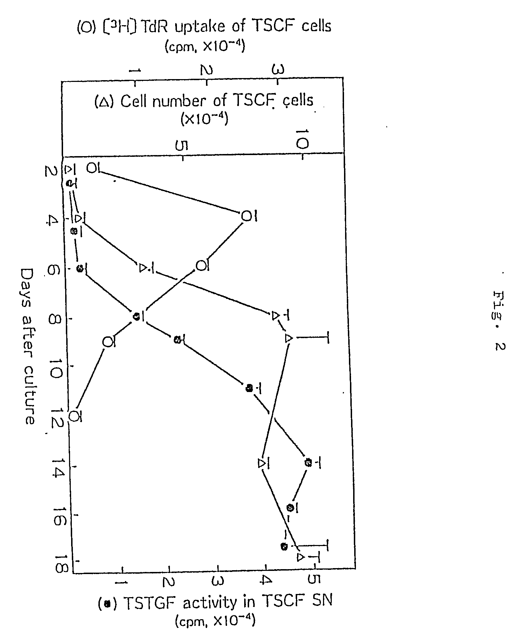

Fig. 2 shows phase-dependent production of TSTGF in TSCF culture SN. TSCF cells (6 x 10³) were cultured in 24-well culture plate in duplicate wells/group, and TSCF cells or culture SN were harvested from one well of each group at days indicated. The number of TSCF cells was counted (Δ - Δ) and TSTGF activity in the culture SN was assayed for the ability to support the growth of 9-16 Th clone (● -●). The uptake of [³H] TdR by TSCF themselves was determined with the use of cells from the other culture well in each group (O - O).

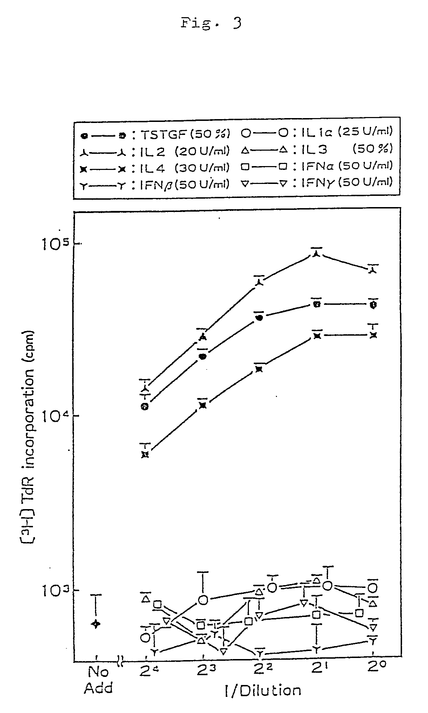

Fig. 3 shows the ability of various lymphokines or monokines to stimulate the proliferation of 9-16 Th clone. 9-16 Th clone cells (1 x 10⁴/well) were cultured with serial dilutions of lymphokines, monokines or interferons in 96-well flat-bottomed microplates for 2 days. Values in the parentheses in the inset indicate the highest concentration of various recombinant materials. Dilution numbers of measuring material from the highest concentration-sample are indicated on abscissa.

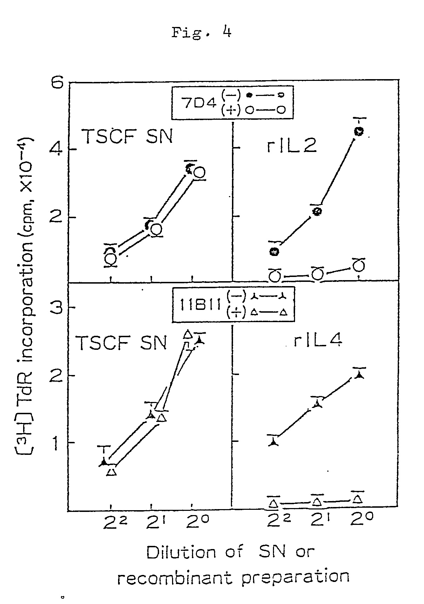

Fig. 4 shows effect of anti-IL2 receptor or anti-IL4 antibody on TSTGF-, IL2- or IL4-induced proliferation of Th clone. 9-16 Th clone (1 x 10⁴ cells/well) was cultured with TSCF-SN or rIL2 (upper panels) or with TSCF-SN or rIL4 (lower panels) in the presence (● or ▲) or absence (O or Δ) of anti-IL2 receptor (7D4) or anti-IL4 (11B11) monoclonal antibody. The highest concentrations used of TSTGF (TSCF SN), rIL2 and rIL4 were 12.5%, 1.25 U/ml and 25 U/ml respectively. The final concentration of ascites containing anti-IL2 receptor (7D4) or anti-II4 (11B11) monoclonal antibody was 1:200 or 1:2000.

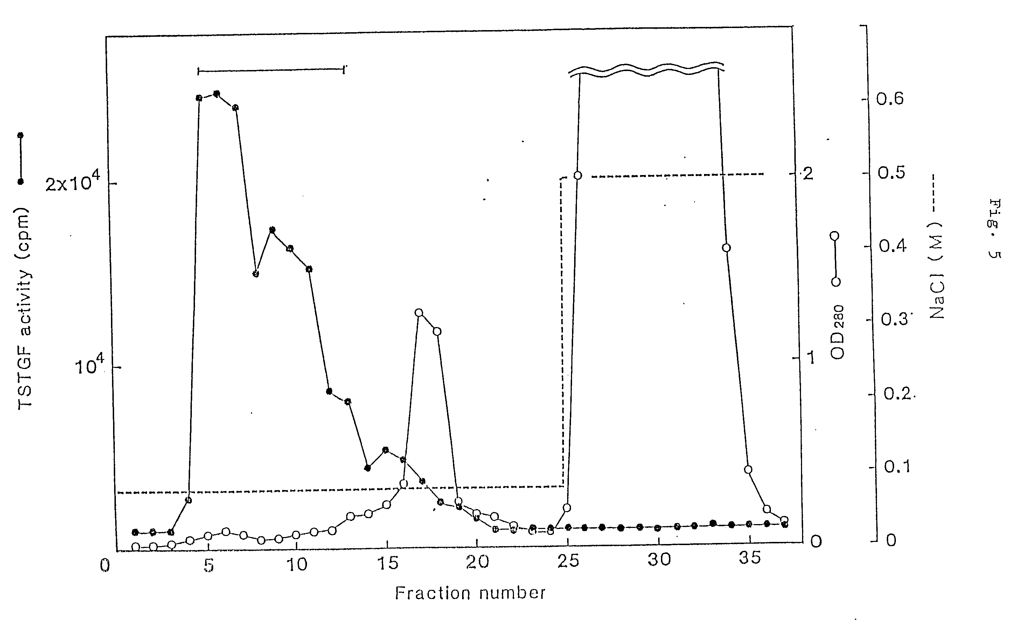

Fig. 5 shows an elution pattern of TSTGF activity from DEAE-Sephacel column. Approximately one liter of TSCF culture SN was dialyzed against 25 mM imidazole-HCl buffer (pH 7.5) and applied to a DEAE-Sephacel column equilibrated with the same buffer. The sample was eluted with 25 mM imidazole-HCl buffer (pH 7.5) containing 0.08 M NaCl and fractions of 50 ml each were collected. Protein concentration is shown as absorbance at 280 nm (O --- O). TSTGF activity (● --- ●) in each fraction was measured as described in Example. Salt concentration is shown by ---. The fractions indicated by the horizontal bar were pooled and used for the next step of purification.

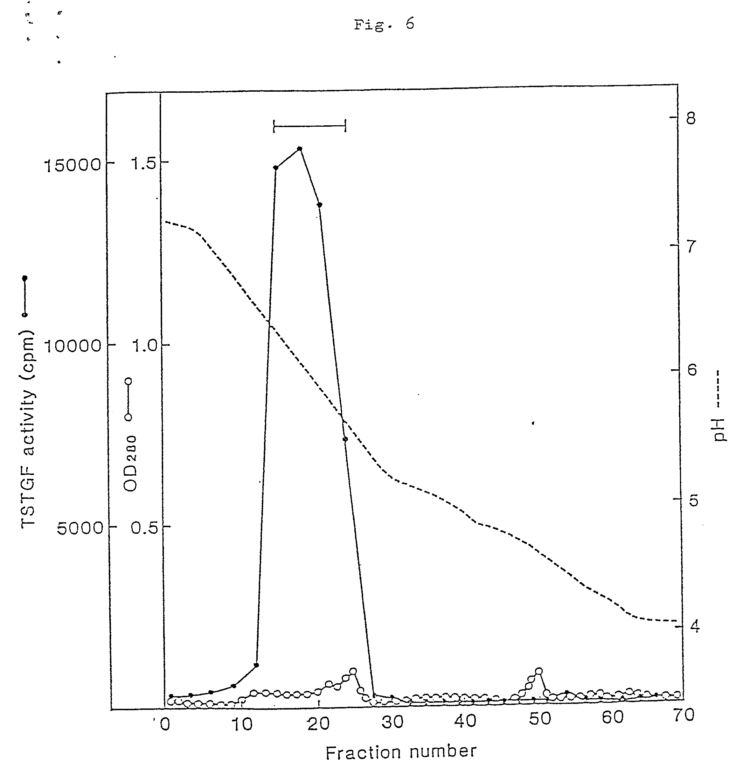

Fig. 6 shows an elution pattern of TSTGF activity from chromatofocusing. Fractions pooled from DEAE-Sephacel column were dialyzed against 25 mM imidazole-HCl buffer (pH 7.5) and loaded on PBE 94 chromatofocusing column. The gradient was generated by using Pharmacia Polybuffer 74 diluted 1/9 with distilled water (pH 4.0). A gradient between pH 7.5 and 4.0 is shown (---). Each 4 ml fraction was collected and dialyzed against HBSS. Ten fractions under the horizontal bar were pooled as the partially purified TSTGF preparation.

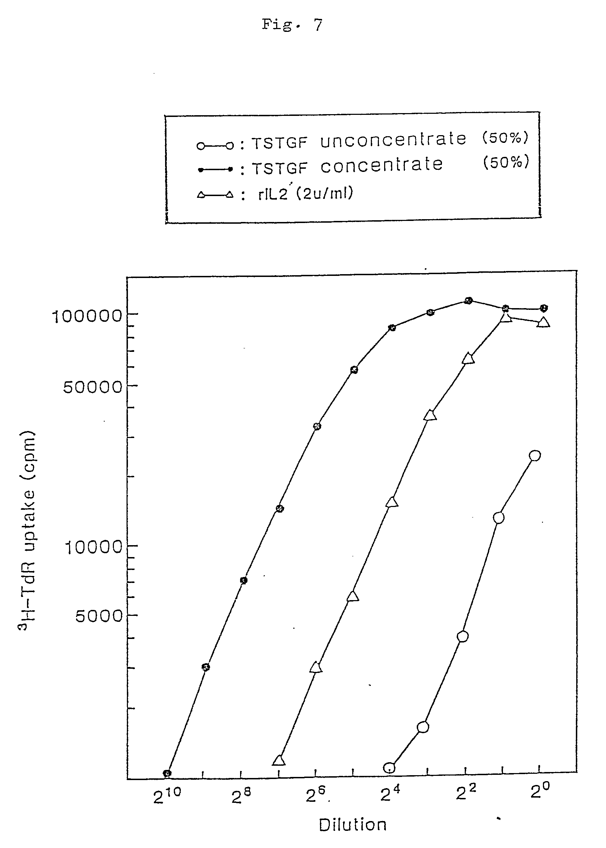

Fig. 7 shows dose response curves of TSTGF activity by partially purified and concentrated TSTGF samples. The partially purified sample obtained from chromatofocusing was concentrated approximately 100-fold. This concentrated (● - ●) or unconcentrated sample (O -O) or rIL2 (Δ - Δ) was titrated for the capacity to induce proliferation of 9-16 Th cells. Values in the parentheses in the inset indicate the highest concentration of samples.

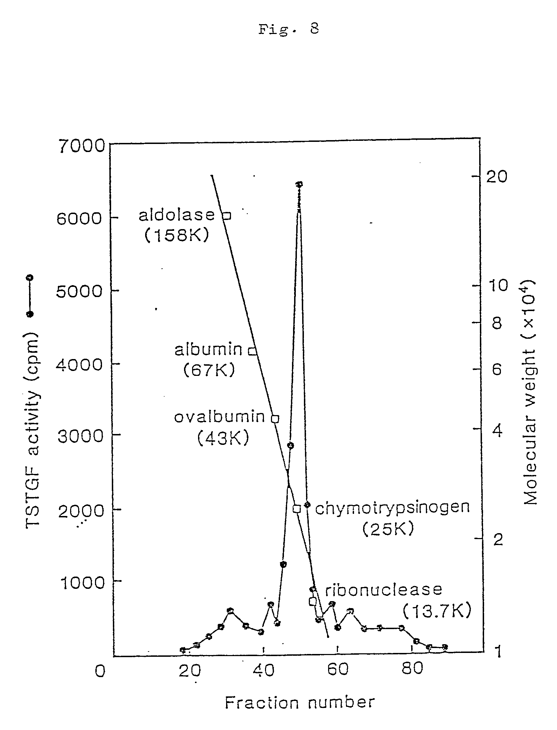

Fig. 8 shows estimation of molecular weight of TSTGF by Sephacryl S-200 column chromatography. The partially purified TSTGF sample was concentrated approximately 100-fold, and applied to a Sephacryl S-200 column in a volume of 0.5 ml. The molecular weights of standards are shown in parentheses.

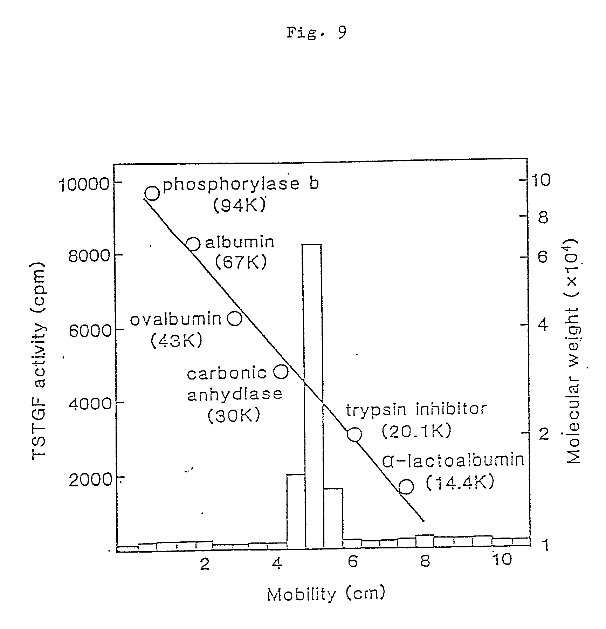

Fig. 9 shows estimation of molecular weight by SDS-PAGE. The partially purified TSTGF sample was concentrated approximately 30-fold, and run in SDS-PAGE with 12.5% acrylamide under nonreducing conditions. Each fraction eluted from 0.5 cm gel slices was assayed for TSTGF activity. The molecular weight standards and molecular weights thereof are shown in the Figure.

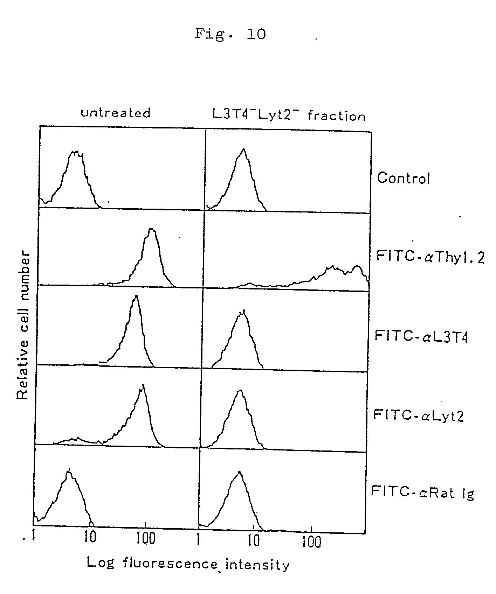

Fig. 10 shows a flow microfluorometry (FMF) analysis of adult thymocytes after depletion of L3T4⁺ and/or Lyt2⁺ cells. Normal C3H/He adult (5-wk-old) thymocytes were treated with a mixture of rat anti-L3T4 and anti-Lyt2 antibodies plus complement (C). The treated cells were then incubated with the above antibodies and after washing, they were incubated on petri dishes which had been pretreated with goat anti-mouse Ig (cross-reactive with rat Ig). The nonadherent cells were collected by gentle pipetting and used as L3T4⁻ Lyt2⁻ fraction.

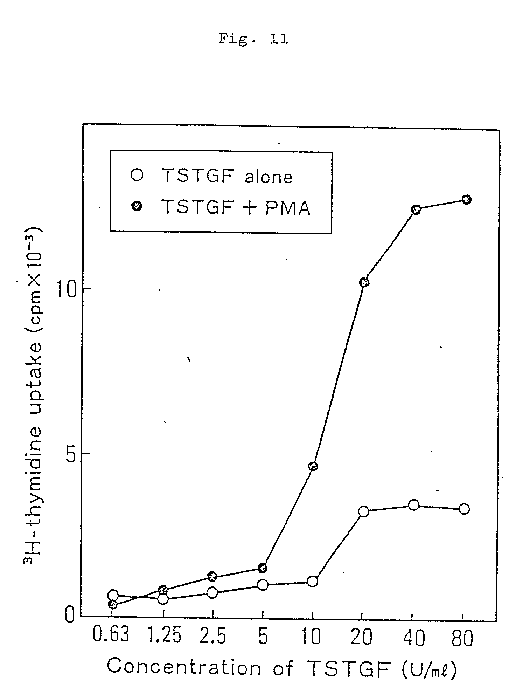

Fig. 11 shows a dose-dependent growth-promoting effect of TSTGF on L3T4⁻Lyt2⁻ thymocytes. Double negative adult thymocytes (3 x 10⁴/well) were cultured for 3 days with various doses of TSTGF alone or together with PMA.

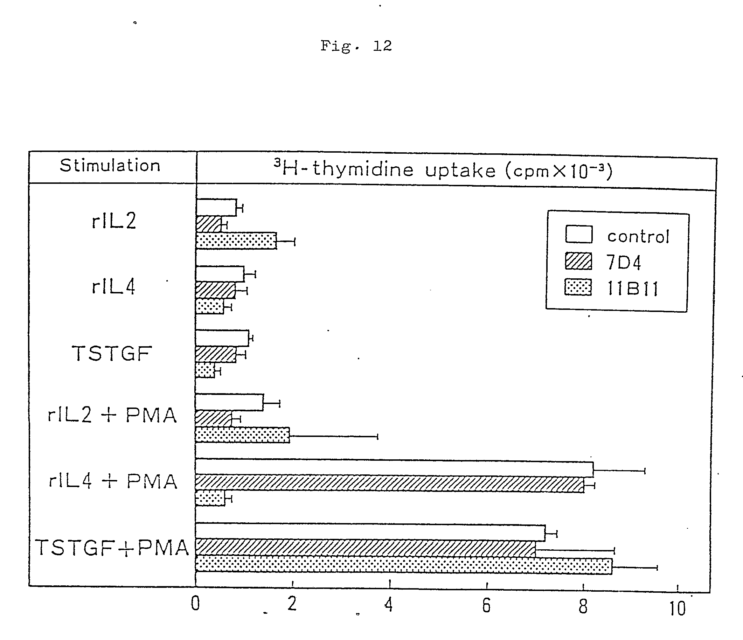

Fig. 12 shows a failure of anti-IL2 receptor or anti-IL4 antibody to inhibit the proliferation of double negative thymocytes induced by TSTGF plus PMA. Double negative (L3T4⁻ Lyt2⁻) thymocytes (3 x 10⁴/well) were cultured with either TSTGF (20 U/ml), rIL2 (2 U/ml) or rIL4 (30 U/ml) for 3 days in the absence or presence of PMA (10 nM). Either anti-IL2 receptor (7D4) or anti-IL4 antibody (11B11) was added to the culture. The final concentration of ascitic fluid containing 7D4 or 11B11 monoclonal antibody was 1 : 200 or 1 : 2000.

3

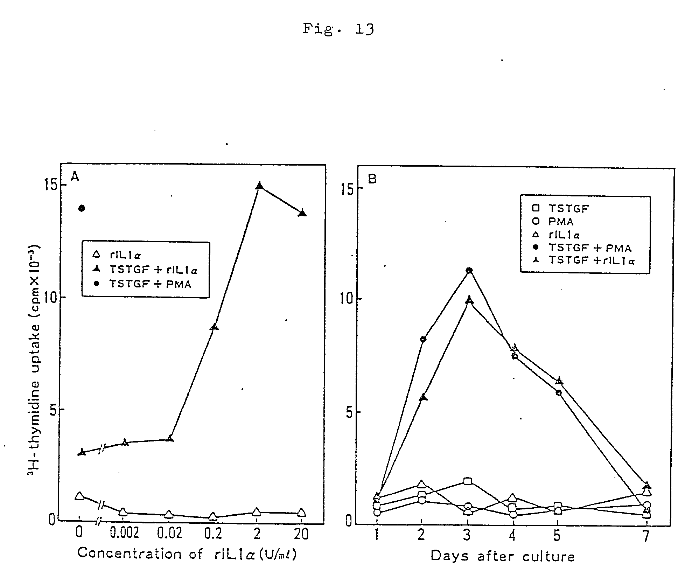

Fig. 13 shows a synergistic effect of TSTGF plus IL1 on the growth promotion of double negative thymocytes. Panel A: Double negative cells were cultured with TSTGF (20 U/ml) plus different concentrations of rIL1α for 3 days. ● indicates the proliferation of double negative cells by stimulation with TSTGF plus PMA. Panel B: Double negative cells were cultured with TSTGF (20 U/ml) plus rIL2 (2 U/ml) for various days.

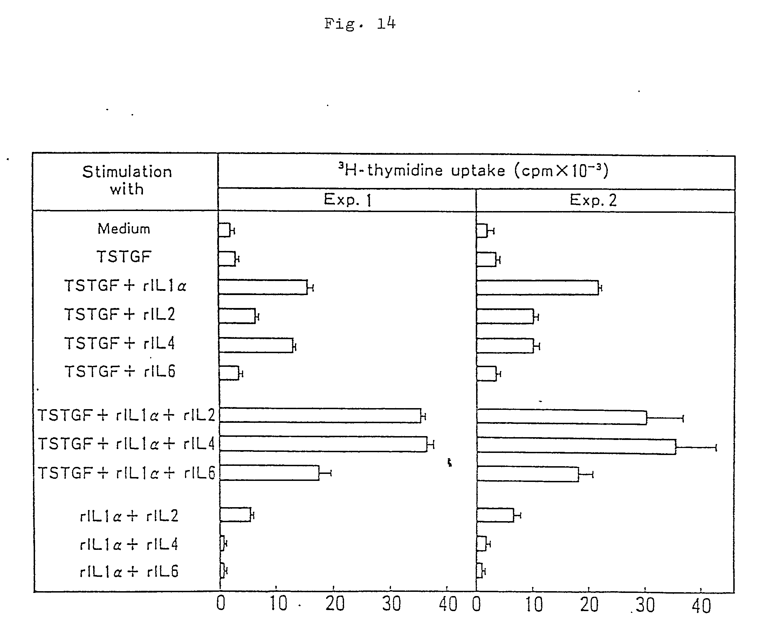

Fig. 14 shows an enhanced proliferation of double negative thymocytes by a combination of (TSTGF + IL1) and IL2 or IL4. Double negative thymocytes were stimulated with combinations of various cytokines for 3 days.

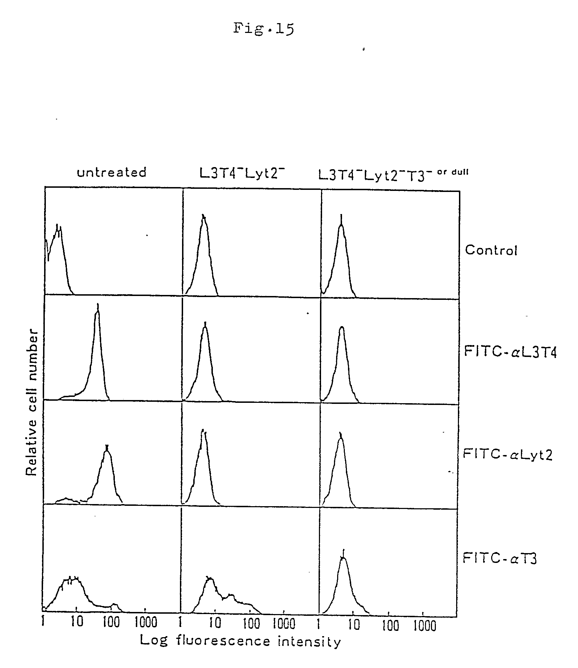

Fig. 15 shows an isolation of L3T4⁻Lyt2⁻T3⁻ or dull adult thymocytes. L3T4⁻Lyt2⁻T3⁻ or dull thymocyte population was prepared by using anti-T3 antibody along with anti-L3T4 and Lyt2 antibodies in the complement treatment and panning steps and by adding goat anti-hamster IgG in treatment of plates for panning.

The cloned thymic stroma cell line TSCF recently developed by us supported the proliferation of the IL2-dependent, antigen-specific helper T cell (Th) clone 9-16 without requiring interleukin (IL)2 and antigen. Such proliferation was also induced by a factor produced in the culture supernatant of the aforesaid thymic stroma cell line TSCF. This substance, namely thymic stroma cell-derived T cell growth factor (TSTGF), could induce proliferation of various T cell clones. The action, brought about by TSTGF, of accelerating the proliferation of T cell is dose-dependent, and could induce the proliferation of T cell while retaining the antigen specificity of the T cell clone. Meanwhile, no IL1, IL2, IL3, IL4, IL5 or interferon activity was detected in the culture supernatant of the thymic stroma cell line TSCF. The proliferation of 9-16Th clone was stimulated by recombinant IL2 and IL4 as well as TSTGF, but not by IL1, IL3, or interferons. However, the proliferation of this Th clone by IL2 or IL4 was almost completely inhibited by anti-IL2 receptor or anti-IL4 monoclonal antibody, respectively. However, the TSTGF-induced proliferation of 9-16Th clone was not affected at all by any of these antibodies. This indicates that TSTGF is functionally different from IL2 and IL4 which represent the major factor with T cell growth activity. In addition, TSTGF activity was also obtained from the supernatant of the primary explant of thymic stroma. Thus, the TSTGF mentioned here is a novel physiologically active substance.

In the present invention, the T cell growth factor TSTGF in the culture supernatant (SN) of the cloned fibroblastic thymic stroma cell line was purified with DEAE-Sephacel chromatography and PBE 94 chromatofocusing, and as a result, its properties have been determined. Namely, the original culture supernatant did not show any of IL1, IL2, IL3, IL4, IL5 and interferon activities, but it contained a considerable amount of IL6 and colony-stimulating factor (CSF) activity in addition to T cell growth factor (TSTGF) activity. However, no IL6 and colony-stimulating factor of any type were detected in the purified preparation of TSTGF. Also, from the chromatofocusing of the T cell growth factor, it was shown that this factor has an isoelectric point (pI) of about 6.0. Besides, when a sample containing the T cell growth activity was applied to Sephacryl S-200 column chromatography and sodium dodecyl sulfate (SDS)/polyacrylamide gel electrophoresis, the T cell growth activity was eluted as a single peak around the molecular weight (m.w.) of about 25,000. Moreover, it was shown that although this T cell growth activity is relatively stable against heat treatment and a wide range of pH, its activity is lost completely by treatment with trypsin or dithiothreitol (DTT). From these results, it is concluded that the TSTGF, namely the new thymic stroma-derived T cell growth factor, is a protein with a molecular weight of about 25,000 and pI of 6.0 and having disulfide bonds in its intact molecule which are indispensable to maintain and exhibit its biological activity.

This invention is explained in further detail as follows:

With the establishment of the thymic stroma cell line TSCF, which was used in this invention, it has become possible to conduct in vitro studies regarding such a mechanism as how each component of the thymic stroma functionally acts on the lymphocytes of T cell lineage. In the past, using as indexes the induction of T cell-associated antigens on the cell surface (Biomed. Res. 2:11, 1981) and the reactivity to a mitogen (Proc. Natl. Acad. Sci. USA 80:6005, 1983), various attempts were made to identify the activity substance in the stages of development from immature thymic cells to mature T cells and to clarify its functions. However, no definite conclusions were obtained (Proc. Natl. Acad. Sci. USA 80:6005, 1983, Scand. J. Immunol. 17:1, 1983).

The active substance TSTGF shown in this invention is produced from the cloned thymic stroma cell line (TSCF) and the primary explant of thymic cell, and accelerates the proliferation of various T cell clones. It is a new active substance clearly different from the cytokines reported heretofore. Therefore, the factor designated as TSTGF in this invention is one of the thymic stroma-derived physiologically active substance relating to the differentiation and proliferation of T cells.

In the study as to the nature of TSTGF of this invention, it is especially important to ascertain functional distinction between the TSTGF and various known lymphokines and monokines including IL2 and IL4 having T cell growth activity. As is clear from the following three facts, there are functional differences between TSTGF and lymphokines and monokines known heretofore.

Firstly, neither interleukins (IL1, IL2, IL3, IL4 and IL5) nor interferons activity (IFN-$\alpha$, -$\beta$, and -$\gamma$) of detectable levels were found in the TSCF culture SN containing TSTGF. TSCF SN contained IL6 and colony-stimulating activity; of the colonies obtained by TSCF SN, 90% or more was of macrophage type while L cell culture SN containing stronger macrophage CSF (M-CSF) activity did not show any TSTGF activity. Moreover, IL6 did not exhibit any TSTGF activity in the system herein described. From these, therefore, TSTGF activity is not mediated by IL6 or M-CSF contaminated in the SN. Secondly, the growth of 9-16Th clone was supported by IL2 and IL4, as in well as TSTGF, but the growth promotion mediated by TSTGF was not affected by anti-IL2 receptor or anti-IL4 monoclonal antibody. However, by adding either one of the antibodies to the medium, the proliferation induced by the relevant interleukin was inhibited almost completely. Thirdly, the important differences between TSTGF and IL2 or IL4 were shown by their differential effect on various T cell clones. The TSTGF induced, to various extent, the proliferation of helper T cell clones, but it failed to support the proliferation of various CTL clones such as CTLL-2 and CT6 which are widely distributed. In contrast, all or some CTL clones responded to the stimulation with IL2 or IL4. These results, therefore, clearly indicate that TSTGF is functionally different from the cytokines reported heretofore. Moreover, the TSTGF did not support the proliferation of HT-2 T cell line which is supported not only by IL-2 but also by keratinocyte-derived T cell growth factor (KTGF) (Proc. Natl. Acad. Sci. USA 83:4451, 1986). Thus, TSTGF is also different from KTGF.

The mechanism by which TSTGF functions to promote the growth of T cells is important in defining the properties of this factor. As mechanism by which this factor induces the proliferation of T cells, there are several possibilities. From the present invention, however, some of these possibilities can be excluded.

(1) The 9-16Th clone, the proliferation of which was induced by TSTGF, continued to proliferate in the presence of TSTGF, but could not proliferate in the absence of TSTGF or antigen and IL2. However, when stimulated by antigen (KLH) + I-E$^k$, the clones began to proliferate. Consequently, as a mechanism by which TSTGF induces the growth of 9-16Th clone, it is unlikely that TSTGF induces tumorigenic potential capable of growing even in the absence of antigens and IL2.

(2) Generally, IL2 plays a central role in the proliferation of T cells (Science 224:1312, 1984). Recent studies have revealed that IL4 can also induce the proliferation of some subsets of T cells (J. Exp. Med. 164:580, 1986, Proc. Natl. Acad. Sci. USA 83:5654, 1986, J. Exp. Med. 165:157, 1987). Therefore, a possibility is raised that TSTGF induces the signal transduction in T cells leading to the production of either IL2 or IL4, which functions as the ultimate T cell growth factor (autocrine mechanism). However, neither IL2 nor IL4 activity was detected in cultures in which 9-16Th cells were proliferating after the stimulation with antigens (KLH + I-E$^k$) or TSTGF. Moreover, the addition of anti-IL2 receptor or anti-IL4 antibody to the culture did not affect the TSTGF-induced proliferation even under conditions in which the addition of appropriate concentrations of such antibody to cultures resulted in almost complete inhibition of the growth-promotion induced by exogenous IL2 or IL4. Therefore, it was concluded that TSTGF exerts its effect without inducing the production of IL2 or IL4.

(3) When 9-16 Th clone was cultured together with TSCF monolayer or the primary thymic explants, these cells adhered to each other. This leads us to presume that there are a series of molecular mechanism that strengthens adhesion between thymic stroma cell and T cell clone, to which TSTGF is possibly related. Generally, cell-cell interaction is essential for many immunological functions (Am. J. Anatomy 170:391, 1984, Science 231:402, 1986, Immunology Today 4:237, 1983, J. Exp. Med. 163:247, 1986). For instance, two antigen-independent pathways are involved in the adhesion of CTL to its targets. One is via CD2 (T11) antigen on CTL and LFA-3 antigen on targets, and the other is via LFA-1 antigen (Nature 323:262, 1986). It has recently been demonstrated that the interaction between human thymic stromal cell lines and human thymocytes is mediated by LFA-3 and CD2 molecules (Clin. Res. 34:422, 1986, J. Immunol. 134-358, 1987, J. Exp. Med. 165:664, 1987, J. Immunol. 138:680, 1987). Such interaction results in a marked increase in the expression IL2 receptor on thymocytes and high reactivity with IL-2 (Clin. Res. 34:422, 1986, J. Immunol. 138:680, 1987). Thus, it is likely that the maturation of T lineage cells in the thymus is the result of a series of complex interactions between thymocytes and thymic stromal cells.

Since 9-16 Th clone adheres to TSCF and primary thymic explant monolayer, it is possible that this adhesion utilizes LFA-3 on TSCF cell surfaces and CD2-equivalent molecule on T cells. It is also known that CD2 (T11) molecule itself is one of the receptor molecules involved in pathways of T cell activation. Therefore, it can be said that the activation of T cells is mediated by either T3-Ti molecular complex or CD2 (T11) molecule. The molecule of the former recognizes certain exogenous antigen + MHC complexes and the latter is considered to be a receptor molecule involved in an activating pathway alternative to that mediated by T3-Ti molecule that appears at an early stage of the intrathymic differentiation of T cells (J. Immunol. 134:330, 1985). Therefore, it is possible that the interaction utilizing LFA-3 and CD2 (T11)-equivalent molecules in cultures of thymic stroma cells and Th cells provides a signal responsible for the proliferation of T cells. In such a model of T cell activation, a possibility is considered that soluble LFA-3 molecules shed off from TSCF cell surfaces represent TSTGF activity. However, a hypothesis that TSTGF functions as the ligand for CD2 (T11)-equivalent molecule is not compatible with the results obtained heretofore about the role of CD2 (T11) in the alternative T cell activation pathway. The proliferation of T cells mediated by CD2 molecules takes place via an autocrine pathway involving the production and release of IL2 and its subsequent binding to IL2 receptor. Therefore, the proliferation of T cells mediated by CD2 molecules is blocked by antibodies to the IL2 receptor (Cell. 36:897. 1984). In contrast, as mentioned above, TSTGF displayed its effect of inducing proliferation even under conditions in which IL2 receptor was almost completely blocked. Therefore, TSTGF is not considered to be the LFA-3 molecule itself and the effect of TSTGF is considered to be exerted through a mechanism different from that the proliferation of T cells is brought about by the cellular adhesion involving LFA-3-CD2 molecules.

Thus, it has been made clear in this invention that TSTGF is produced by thymic stroma cells which originally interact with thymocytes in the thymus. It is therefore considered that, through this factor, T cells in the thymus receive a signal for proliferation. Moreover, since IL-2 and IL-4 do not participate in this process, it is postulated that TSTGF contributes to the expansion of T cell progenitors emigrating into the thymus from the bone marrow, before the expression of functional IL2 receptor on thymocytes. In fact, we have recently studied the physiological functions of TSTGF and the mechanism by which TSTGF exerts its effect, and the results have revealed that TSTGF induces the proliferation of various subset cells of thymic T cell partially immature thymocytes in addition to T cell clones disclosed in this specification.

The physicochemical properties of TSTGF of this invention are summarized below:

The supernatant of TSCF culture was fractionated by continuous processes with DEAE-Sephacel and PBE 94 columns. And the fractions containing TSTGF activity were pooled. The sample thus pooled was separated from the contaminated proteins which are inactive and constitute the greater part of the TSCF culture supernatant. As a result, the specific activity increases about 300 times on protein basis. This was taken as partially purified preparation of TSTGF. The original of TSCF supernatant did not show IL1, IL2, IL3, IL4, IL5, or interferon activity of any detectable level, but it contained a considerable amount of IL6 and macrophage CSF activities. However, in the partially purified preparation no IL6 and CSF activities were not detected. Moreover, the partially purified TSTGF did not contain any other CSF of detectable level such as GM-CSF which has recently been reported as capable of inducing the proliferation of HT-2 cell, a helper T cell clone (J. Immunol. 138:4208, 1987, J. Immunol. 138:4293, 1987). From these facts, it was revealed that the partially purified preparation could be used to determine the physicochemical propertied of TSTGF as shown below.

The properties of TSTGF are determined from a series of analyses using the partially purified preparation as follows: (a) According to analyses by Sephacryl S-200 chromatography and SDS-PAGE, the molecular weight of the TSTGF activity is about 25,000. (Figs. 8 and 9); (b) Isoelectric point (pI) measured by the chromatofocusing is about 6.0 (Fig. 6); (c) Stable against each treatment with heat (65°C, 10 min.) and with pH 2.0 - 9.0 (Table 6); (d) Highly susceptible to each treatment with trypsin and DTT (Table 7), which suggests that the disulfide bond plays an crucial role in the TSTGF activity. Furthermore, when a concentrated sample was used, the maximal stimulation of proliferation of 9-16Th clone was almost comparable or slightly larger than that obtained from rIL2 (Fig. 7). Also from its functional characteristics (Table 4) made clear by its capability of inducing proliferation of various types of T cell clone, TSTGF is different in properties from various factors which have hitherto been reported to relate to the proliferation of T cells (J. Immunol. 138:4208. 1987, J. Immunol. 138:4293, 1987, Immunological Rev. 63:173. 1982, Immunological Rev. 78:185, 1984, Ann. Rev. Immunol. 5:429, 1987, Methods in Enzymology 116:588, 1985, J. Immunol. 138:3314, 1987). These differences in properties are summarized in Table 11. From these facts, it has been concluded that TSTGF is a T cell growth factor clearly different from any known cytokines including the factors listed in Table 11.

Another remarkable point is the biological functions of TSTGF on the promotion of the growth of thymic T lymphocytes. TSTGF virtually acts on fresh thymic lymphocytes as well as established cell lines as mentioned above. Since TSTGF is produced by thymic stroma-derived cloned cell line (TSCF) and primary explant of thymic stroma, it is presumed that TSTGF contributes to differentiation and proliferation of thymic T cells by especially effecting immature thymocytes. However, when the original TSCF culture supernatant was added, either alone or together with phorbol myristate acetate (PMA), to a culture of fresh lymphoid cells (spleen, lymph nodes or thymocytes), any significant proliferation was not induced. In contrast, by the stimulation with purified TSTGF preparation prepared in this invention together with PMA, a potent proliferation of immature thymocytes (L3T4-Lyt-2⁻) of the thymus of an adult mouse as well as of thymocytes of a fetal mouse on 14-day gestation was induced. Since such an enhanced proliferation was not inhibited by anti-IL4 or anti-IL2 receptor antibody, this was again not due to the stimulation of an autocrine mechanism involving the production and utilization of IL4 or IL2. In scrutinizing PMA-equivalent physiological substances, IL1 was revealed to be capable of replacing the role of PMA in the above costimulation cultures and inducing enhanced proliferation of double negative (L3T4⁻ Lyt2⁻) immature thymocytes in combination with TSTGF. Although TSTGF plus IL2 or IL4 also exhibited appreciable or moderate synergistic effect on the growth of such double-negative thymocytes, its magnitude was weaker compared to that obtained by TSTGF plus IL1. More importantly, the strikingly enhanced proliferation was induced in the combination of TSTGF, IL1 and IL2 or IL4 under the condition in which the proliferation induced by IL1 plus IL4 or IL1 plus IL2 was marginal or slight. Furthermore, such strongly enhanced proliferation was also observed in the double negative thymocyte population which was additionally depleted of T3⁺ cells (namely, the L3T4⁻ Lyt2⁻ T3⁻ or dull population). These results indicate the crucial role of TSTGF in the proliferation of immature thymocytes by synergy with various cytokines.

It has recently been reported that a similar activity is present in IL4 (EMBO J. 6:91, 1987). As described above, however, no IL4 activity was detected in the purified TSTGF preparation and TSTGF is apparently distinct from IL4 in not only physicochemical but also functional properties.

The above is summarized as follows: The TSTGF of this invention is a new T cell growth factor having physicochemical and functional characteristics clearly different from those of any known cytokines which exert T cell growth activity. And the TSTGF derived from thymic stroma cells is considered to play an important role in the differentiation and proliferation of the lymphocytes of T cell lineage which emigrated into the thymus. In addition, the TSTGF of this invention promotes the proliferation of T cells including immature thymocytes. Therefore, the TSTGF in this invention can be applied to immunotherapy for diseases, such as AIDS, resulting from immunodeficiency and for various infections and malignant tumors.

Main abbreviations used in this specification are as follows:

IL = interleukin; TSTGF = thymic stroma-derived T cell growth factor; Th = helper T cell; KLH = keyhole limpet hemocyanin; Con A = concanavalin A; SN = supernatant; INF = interferon; TSCE = thymic stroma cell line in epithelial form; TSCF = thymic stroma cell line in fibroblastic form.

The examples illustrate the invention.

## Example 1

### Mice

Female C3H/HeN and BALB/c mice, 6 - 8 weeks of age, were purchased from Charles River Laboratory (Japan), Kanagawa, Japan and Shizuoka Experimental Animal Laboratory, Shizuoka, Japan, respectively.

### T cell clones

Three helper T cell (Th) clones, 9-16, 8-E and 8-5 (specific for KLH and restricted to I-Eᵏ (9-16) or restricted to I-Aᵇ (8-E and 8-5)) were used. Other two Th clones are autoreactive anti-I-Aᵇ (GLT2) and anti-I-Eᵏ (BR2) Th clones. IMP clone is an anti-Kᵏ allo-CTL clone. These T cell clones were maintained by a modification of the method described by Kimoto and Fathman (J. Exp. Med. 152:759, 1980), namely by repeating alternating cycles of 7-day antigenic stimulation followed by 7-day rest without stimulation in the presence of culture supernatant (Con A SN) of murine concanavalin A (Con A)-stimulated lymphocytes, with X-ray-irradiated spleen cells as feeder and with addition of KLH, if necessary. Other three IL2-dependent cell lines, CTLL-2 (J. Exp. Med. 149:273, 1979), CT6 and HT-2 (J. Exp. Med. 150:1510, 1979) were maintained in cultures containing murine Con A SN.

Thymic stroma cell line

We have recently established two types of cell lines from culture BALB/c thymic stroma tissue cultures. Morphologically, one displays epithelial characteristics, while the other displays fibroblastic characteristics. In this specification, these two types of cell lines are abbreviated as TSCE (thymic stromal cell line in epithelial form) and TSCF (thymic stromal cell line in fibroblastic form), respectively. TSCE and TSCF were tested periodically with the mycoplasma test system (Gen-Probe, San Diego, CA) and were used after confirming no mycoplasma contamination.

Primary monolayer culture of thymic stroma cells

The primary monolayer culture of thymic stroma cells was conducted by a modification of the conventional method (Proc. Natl. Acad. Sci. USA 80:6005, 1983). Briefly, 1 - 2 x $10^8$ cells of C3H/He thymocytes were cultures in a 25-cm² culture flask in 10 ml of culture medium described below. Two days after start of culturing, thymocytes were gently washed away, and scattered few adherent cells were fed with 50% fresh medium and 50% conditioned medium obtained after removing, by centrifugation, thymocytes from the primary thymic explant culture. Thereafter, the primary cultures were maintained by weekly feeding with a similar 50:50 mixture of fresh and conditioned media.

Harvest of culture supernatants containing thymic stroma-derived T cell growth factor (TSTGF)

The supernatants were harvested from cultures of established thymic stroma cell lines or primary monolayer cultures of thymic stroma cells one week after the monolayer reached confluency.

Culture medium

All T cell clones were maintained in RPMI1640 supplemented with 10% fetal bovine serum (FBS) (Armour Pharmaceutical Co., Kankakee, IL), 5 x $10^{-5}$ M 2-mercaptoethanol (2-ME) and streptomycin (100 μg/ml) penicillin (100 U/ml). In order to maintain established thymic stromal cell lines and to establish primary monolayers of thymic stroma cells, Dulbecco's minimal essential medium (DMEM) supplemented with 10% FBS, 5 x $10^{-5}$ M 2-ME and gentamycin (50 μg/ml) was used. This DMEM complete medium was also used for all types of proliferative responses.

Monoclonal antibodies

Anti-IL2 receptor (7D4) and anti-IL4 (11B11) monoclonal antibodies, described in Proc. Natl. Acad. Sci. USA 80: 5694, 1983, and Nature 315:333, 1985, respectively, were used.

Proliferative responses of Th cell clones

Step-wise dilutions (100 μl) of test samples (culture supernatants of thymic stroma cell lines) were added to a 1 - 5 x $10^5$ cells/ml Th clone suspension (100 μl) in wells of flat-bottomed 96-well microplates (No. 25860: Corning Glass Works) and incubated at 37°C in CO₂ incubator (95% air / 5% CO₂) for 2 days, unless otherwise indicated. After that, the cells were pulsed with 0.5 μCi/well of [³H] thymidine for 7 hr and then harvested onto a glass filter with an automated sample harvester (Bellco Glass Inc., Vineland, NJ), and the radioactivity incorporated into the DNA was measured with a liquid scintillation spectrometer (Aloka Co., ltd., Tokyo).

IL1 assay

The assay to detect IL1 activity with the use of the T cell clone LBRM-33 1A5 that produces IL2 in the presence of IL1 and phytohemagglutinin (PHA) is described in Proc. Natl. Acad. Sci. USA 78:1133, 1981. Briefly, $1 \times 10^5$ of LBRM-33 1A5 cells were cultured in 200 $\mu$l of assay medium for 20 hr at 37° C in flat-bottomed 96-well microplates. This medium contains PHA (2 $\mu$g/ml) and step-wise diluted standard IL1 or test sample. After culturing, cell-free SN was collected from each well and tested for IL2 activity in an IL2 assay system (described below) using IL2-dependent T cell line CTLL-2. By thus measuring IL2 activity, IL1 activity in the test sample was determined.

IL2 assay

IL2 activity was assayed by measuring [³H] thymidine uptake of IL-2-dependent murine T cell line CTLL-2 (J. Exp. Med. 152:759, 1980). For the assay, $1 \times 10^4$ CTLL-2 cells were cultured in a volume of 200 $\mu$l containing test samples, with different concentrations of recombinant murine IL2 as standard. After 24 hr of incubation at 37° C, cells were pulsed with 0.5 $\mu$Ci/well of [³H] thymidine (TdR) and cells were harvested 8 hr later.

IL3 assay

IL3 activity was assayed by measuring the [³H] thymidine uptake of IL3-dependent cell line FDC-P2 (J. Exp. Med. 152:1036, 1980). For the assay, $2 \times 10^4$ FDC-P2 cells were cultured in 200 $\mu$l of a culture solution containing different dilutions of the recombinant murine IL3 or test samples. After 24 hr of incubation, cells were pulsed with 0.5 $\mu$Ci/well of [³H] TdR and harvested 4 hr later.

IL4 assay (B cell costimulation assay)

IL4 (BSF-1) activity was measured by a modification of the anti-IgM costimulation assay method (J. Exp. Med. 155:914, 1982). Briefly, BALB/c splenic B cells were prepared by the generally known method (J. Exp. Med. 154:1681, 1981, J. Immunol. 130:2219, 1983), and $10^5$ purified B cells were cultured for 3 days with various dilutions of recombinant murine IL4 or test samples with or without anti-IgM at 5 $\mu$g/ml. DNA synthesis was assessed by a final 16-hr pulse with 1 $\mu$Ci/well of [³H] TdR.

Results

Growth response of Th clone by a fibroblastic form of thymic stroma-derived cell line or its culture SN

· Two types of BALB/c thymic stroma-derived cell lines -TSCF (fibroblastic form) and TSCE (epithelial form)- were recently established by us. To examine the functional activities of these cell lines in their interaction with T cells, we first tested the ability to support the growth of T cell clones. The 9-16 Th clone, a KLH-specific and I-E^k restricted Th clone (Thy-1⁺, Lyt-1⁺, Lyt-2⁻) which was established from KLH-primed (C57BL/6 x C3H/He) Fl spleen cells (J. Exp. Med. 158:1178, 1983) was co-cultured with TSCF, TSCE or BALB/3T3 monolayer. As shown in Expt. 1 of Table 1, the yield in cell number of 9-16 Th clone co-cultured with TSCF monolayer for 9 days showed an approximately 3-5 fold increase as compared with the initial input cell number in the absence of KLH and I-E^k antigen-presenting cells or exogenous lymphokines. This was in contrast to a decrease in cell number of 9-16 Th clone cultured alone or co-cultured with TSCE or BALB/3T3. In addition, the ability of TSCF monolayer to support the growth of 9-16 Th clone was also found in culture SN of TSCF (Expt. 2 of Table 1). Such growth of 9-16 Th clone in the presence of culture SN of TSCF was also proved by [³H] thymidine (TdR) uptake and the peak response of DNA synthesis was observed 2 - 3 days after cultures of Th clone with TSCF culture SN (Fig. 1). These results indicate that the thymic stroma-derived cell line which was established recently and abbreviated as TSCF in this specification has the capability to support the growth of Th clone without requiring KLH and I-E$^k$ antigen-presenting cells or exogenous lymphokines. Moreover, it produces, in the culture supernatant, a soluble factor of factors capable of supporting such growth. Thus, the said factor was named as thymic stroma-derived T cell growth factor (TSTGF).

### The optimal timing of harvesting TSCF culture to obtain potent TSTGF activity

To determine the optimal timing of obtaining potent TSTGF activity from TSCF cultures, TSCF cells and their culture SN were harvested from the one well of duplicate TSCF cultures on various days after seeding in 24-well plate. And the number of TSCF cells harvested and the TSTGF activity in the harvested culture SN were measured. The DNA synthesis of TSCF themselves was measured by [$^3$H] TdR uptake in the other well of duplicate cultures. The results are summarized in Fig. 2. The growth of TSCF themselves as evaluated by the increase in cell number continued until around day 8 of the culture when TSCF formed a confluent monolayer. Meanwhile, the peak response of [$^3$H] TdR uptake by TSCF preceded the increase in the number of these cells. The peak was seen on day 4 of the culture, and began to decrease after the cell monolayer acquired confluency. The TSTGF activity in TSCF culture became detectable around day 8 or 9 concurrently with the acquisition of confluence by TSCF monolayer. Thereafter, the activity continued to increase, and the maximum activity was obtained 12 - 14 days after seeding. Thus, the TSCF culture SN harvested at this timing was used as a source of TSTGF in the following experiments.

### Retention of antigen-reactivity of Th clone after maintenance of its growth in culture supernatant containing TSTGF

Next we studied as to whether Th clone maintained with addition of TSTGF would retain its antigen-reactivity. 9-16 Th clone was cultured either on TSCF monolayer or with TSCF culture SN (TSTGF) for 4 weeks by weekly change of culture medium, and tested for the reactivity to stimulation with KLH + I-E$^k$ antigen-presenting cells (Table 2). The results demonstrate that the 9-16 Th clone harvested after culturing on TSCF monolayer or with TSTGF did not acquire capability to proliferate spontaneously like malignant transformed cells but clearly retained antigen (KLH)-specific reactivity. Therefore, even in the absence of antigen$^+$ antigen-presenting cells, the Th clone proliferation is promoted by TSTGF, while retaining its antigen-reactivity.

### Lymphokine and monokine activities in culture SN of TSCF

A number of lymphokines and monokines produced by lymphocytes and monocytes have so far been reported. Therefore, a study was made as to whether any known lymphokine or monokine activity can be detected in culture SN of TSCF monolayer. Recombinant lymphokines and standard culture SNs containing lymphokines were used as positive control in the corresponding assay systems. And by comparing them with culture SN of TSCF, IL1, IL2, IL3, IL4 and IL5 activities therein were tested. A representative result summarized in Table 3 shows that positive controls of IL1, IL2, IL3 and IL4 functioned to proliferate or activate the corresponding responding cells, whereas culture SN of TSCF did not show such interleukin activity of detectable level. It was further shown by other experiments that IL5 and interferon (IFN) activities were not detected in TSCF SN.

### Relationship between TSTGF and various interleukins

In order to further elucidate the relationship between TSTGF and lymphokines which have been known as T cell growth factor, a study was first made as to which type or types of lymphokine are capable of promoting the proliferation of 9-16 Th clone. Interleukins (IL1, IL2, IL3 and IL4) and interferons (IFN-$\alpha$, IFN-$\beta$ and IFN-$\gamma$) were tested for their ability to support the growth of 9-16 Th clone in comparison with that obtained by TSTGF. The results in Fig. 3 demonstrate that the proliferation of 9-16 Th clone was induced by IL2 and IL4 as well as TSTGF but not by IL1, IL3 and interferons. Moreover, another experimental results also revealed that IL5 and IL6 did not induce the proliferation of 9-16 Th clone. Althouth IL2 or IL4 activity was not detected in culture SN from TSCF monolayer (Table 3), the results in Fig. 3 present a possibility that the growth of 9-16 Th clone was promoted by IL2 or IL4 produced in undetectable quantities in TSCF culture SN. This possibility was investigated by examining the effect of anti-IL2 receptor or anti-IL4 monoclonal antibody on TSTGF-induced proliferative responses of 9-16 Th clone.

The results in Fig. 4 demonstrate that the proliferation of 9-16 Th clone was supported by IL2 and IL4 (right-hand-side panels) and that such proliferation was almost completely inhibited by the addition of anti-IL2 receptor or anti-IL4 antibody. In contrast to this, TSTGF-induced proliferation of 9-16 Th clone was little affected by the existence of any of these antibodies (left-hand-side panels). These results indicate that TSTGF induced the proliferation of Th clone in a mechanism which is entirely different from that in IL2 or IL4 and that TSTGF is functionally different from IL2 and IL4.

## Characteristics of TSTGF on proliferation of Th and CTL clones

Next, a study was made on the effect of TSTGF on the promotion of the proliferation of various Th and CTL clones. As shown in the results in Table 4, TSTGF was capable of proliferating various but not all types of Th clones (each different in antigen specificity and restriction specificity to major histocompatible antigen (MHC)), though in varying degree of proliferation in each clone. On the other hand, TSTGF failed to support the proliferation of newly established CTL clones such as anti-$K^k$ (IMP) clone used in this experiment and also of various CTL clones used widely heretofore.

Meanwhile, IL2 induced the proliferation of all T cell clones, and IL4 induced the proliferation of a part of Th and CTL clones. Thus, the kinds of T cell clones exhibiting TSTGF-induced proliferation were different from those obtained by IL2 or IL4. This leads us to the conclusion that TSTGF is functionally different from IL2 or IL4.

## Growth promotion of Th clone by the primary thymic explant culture cells

A C3H/HeN thymic cell suspension containing both thymocytes and thymic stromal cells was seeded on culture flasks. Adherent cells were cultured after discarding non-adherent cells (mainly thymocytes and dead cells) and fed with 50% fresh medium and 50% conditioned medium. When 9-16 Th clone was cultured with TSCF, TSCE or primary adherent thymic explant cell layer, the adherent cells obtained from the primary thymic cultures were similar to TSCF cell line not only in morphological characteristics but also in the ability to support the proliferation of Th clone. And 9-16 Th cells adhered to TSCF and primary thymic culture cells and proliferated though to different extent. Importantly, it has become clear that the primary thymic explant culture cells also produce TSTGF activity and this TSTGF exhibits growth-promotion selectively on Th clone (Table 5). Therefore, this result indicates that TSTGF capable of supporting the proliferation of T cell clone is produced by primary thymic explants as well as by thymic stroma-derived cell lines.

## Example 2

In order to study the physicochemical properties of TSTGF which is obtained from the culture supernatant of the thymic stroma-derived cell line, the following experiments were conducted.

## Thymic stroma-derived cell line

As mentioned above, thymic stroma-derived cell line TSCF was established by us. Morphologically, this cell line has a fibroblastic appearance. In the following experiments, a cloned cell line of this TSCF was used.

## Measurement of TSTGF activity

Test samples (crude or partially purified TSTGF) (100 $\mu$l) were added to wells of 96-well flat-bottomed microplates (No. 25860: Corning Glass Works) to which 100 $\mu$l of 9-16 Th clone suspension (1 x $10^5$ cells/ml) had been placed and incubated in a $CO_2$ incubator (95% air/5% $CO_2$) for 2 days at 37° C. Then, these cells were pulsed with 0.5 μCi/well of [³H] thymidine (New England Nuclear, Boston, MA) and 7 hr. later harvested onto the glass filter with an automated sample harvester (Bellco Glass Inc., Vineland, NJ), and the incorporated radioactivity was measured with a scintillation spectrometer (Aloka Co., Ltd., Tokyo, Japan).

Measurement of colony-stimulating factor (CSF) activity

In the measurement of proliferative responses to CSF, two different methods were used. These are essentially the same as the known methods (J. Immunol. 131:2374, 1983). The one is a classical method in which colony formation in semisolid agar is measured. The other is a method based on uptake of [³H] thymidine (³H-TdR) by bone marrow cells stimulated by CSF. The basic cloning assay of CSF is detailed in Infect. Immunn. 26:408, 1979. Briefly, 10⁵ nucleated femoral bone marrow cells per ml were suspended in warm (42°C), enriched McCoy's 5A medium containing 15% FBS, antibiotics, and 0.3% agar. One ml of this mixture was added to a 35-mm petri dish containing up to 0.25 ml of a CSF source. After cooling, incubation was performed in 5% $CO_2$ at 37°C for 10 days. The dish was then inspected with a microscope at 35x magnification, and the proliferative centers ($\geq$ 25 cells/colony) were scored. The ³H-TdR incorporation assay method developed for measuring CSF activity is basically a modification of the existing assay methods for interleukin 1 (J. Immunol. 116:1466, 1976) and interleukin 2 (Mol. Immunol. 17:579, 1980) with the exceptions that the number of responding cells is different and an exogenous primary mitogenic signal is not required. In this system, no additives other than CSF were added. Briefly, 0.5 -1 x 10⁵ nucleated femoral bone marrow cells obtained by separation of a mouse density centrifugation medium (Lympholyte M. Cedarlane Laboratories Ltd., Ontario) were incubated in a total volume of 0.2 ml in individual wells of 96-well plates containing CSF. The medium used and incubation conditions were the same as those employed in the cloning assay, with the deletion of semisolid agar. The incubation was performed for a total of 72 hr with the addition of 1 μCi of ³H-TdR in the last 6 hr. The cultures were then harvested onto the glass filter with a cell harvester and ³H-TdR incorporation was measured by liquid scintillation spectrometry.

DEAE-Sephacel chromatography

The culture supernatant containing TSTGF activity was dialyzed against 25 mM imidazole-HCl buffer (pH 7.5) and applied to a column of DEAE-Sephacel (3.0 x 45 cm) equilibrated with the same buffer. The column was washed with 1000 ml of the same buffer and eluted with 25 mM imidazole-HCl buffer (pH 7.5) containing 0.08 M sodium chloride at a flow rate of 20 ml/hr. Fractions of 50 ml each were collected and dialyzed against Hanks balanced salt solution (HBSS), and measurements were made on TSTGF activity and protein content by O.D. 280 nm. The protein remaining in the column was further eluted with 25 mM imidazole-HCl buffer (pH 7.5) containing 0.2 M sodium chloride. Then, measurement was made on the activity in a similar manner.

PBE 94 chromatofocusing

Chromatofocusing was performed using PBE 94 column. Briefly, the PBE 94 column (1.0 x 25 cm) was equilibrated with 25 mM imidazole-HCl buffer (pH 7.5), and the sample which had been dialyzed against the same buffer was loaded on the column. The column was washed with 20 ml of the same buffer before the gradient was begun. The gradient was produced by using Pharmacia Polybuffer 74 diluted 1/9 with distilled water (pH 4.0) at a flow rate of 16 ml/hr (Thus, pH gradient was produced between 7.5 and 4.0). Fractions of 4 ml each were collected, dialyzed against HBSS, and used for measurement of the activity.

Sephacryl S-200 gel filtration chromatography

TSTGF activity-containing fractions obtained from PBE 94 column was concentrated 10-fold by ultrafiltration by using an Amicon 8400 cell YM10 membrane (Amicon corp., Lexington, MA). The concentrate was then dialyzed against HBSS buffer. Two ml of concentrated sample was applied to a 1.5 x 9.5 cm Sephacryl S-200 (Pharmacia Fine Chemicals) column equilibrated with the same buffer. Fractions (2.5 ml) were collected, dialyzed with HBSS, and assayed for TSTGF activity.

Sodium dodecyl sulfate (SDS)-polyacrylamide gel electrophoresis (PAGE)

The TSTGF-containing concentrated sample after chromatofocusing was analyzed by discontinuous SDS-PAGE (0.75 mm x 16 cm x 20 cm slab gel) with an upper gel (1 cm) consisting of 2.88% acrylamide, 0.12% bis-acrylamide, 0.125 M Tris-HCl (pH 6.8) and 0.1% SDS, and a lower gel (8 cm) consisting of 11.52% acrylamide, 0.48% bis-acrylamide, 0.375 M Tris-HCl (pH 8.8) and 0.1% SDS (Methods in Enzymol. 116:493, 1985). The running buffer contained 0.025 M Tris base, 0.192 M glycine and 0.1% SDS. The gel was run at 30 V until the sample entered the lower gel (about 1 h) and then at 120 V until completion (about 4 hr). After electrophoresis, the SDS-PAGE gels were sliced into 5 mm fragments. Individual fragments were eluted with 1 ml of RPM11640 medium containing 10 mg/ml bovine serum albumin, which were then used for measuring TSTGF activity. The gels loaded with the sample or standard proteins were stained by the method of Merril et al. (Science 211:1437, 1981).

Chemical and enzymological treatments of partially purified TSTGF

Fifty $\mu$l sample of partially purified TSTGF obtained by DEAE-Sephacel chromatography and PBE 94 chromatofocusing was treated with trypsin (15 $\mu$g/ml, 37°C, 2 hr) or dithiothreitol (DTT) (50 ml, 25°C, 2 hr). The biological stability of TSTGF was examined by heating and pH treatment using either one of 0.2 M Tris-HCl, sodium acetate or glycine-HCl. After these treatments, the sample were dialyzed against HBSS and used for measuring TSTGF activity.

Results

Partial purification of TSTGF

The TSTGF activity was monitored by the proliferation of helper T cell (Th) clone 9-16. TSCF culture supernatant was collected 1 week after the TSCF cell line acquired confluence. To determine the optimal salt concentration for elution of TSTGF from DEAE-Sephacel column, elution was performed by serially increasing a concentration of sodium chloride. The results showed that the majority of the TSTGF activity was eluted of 0.08 M sodium chloride. Therefore, to achieve partial purification, about 1 ℓ of culture supernatant was applied to DEAE-Sephacel column and eluted with buffer containing 0.08 M sodium chloride. As a result, all TSTGF activity was recovered in this fraction (Fig. 5). It is interesting that the greater part of the TSTGF activity is eluted at a comparatively early stage with 0.08 M sodium chloride and clearly separated from the major peaks of proteins eluted with 0.08 M NaCl and with 0.5 M NaCl.

The TSTGF activity-containing fractions thus obtained from DEAE-Sephacel column were further applied to a PBE 94 chromatofocusing column. A typical elution profile is shown in Fig. 6. The TSTGF activity was eluted as a single peak having pI value of about 6.0. Also, when TSCF culture supernatant without being applied to DEAE-Sephacel column was applied directly to PBE 94 chromatofocusing in a small scale, TSTGF activity was similarly eluted as a single peak with pI of 6.0. TSTGF activity-containing fractions obtained by separating DEAE-Sephacel-fractions and the chromatofocusing were pooled and used as a partially purified TSTGF preparation for the following experiments. The recovery ratio of TSTGF activity after being applied to DEAE-Sepharose and chromatofocusing columns was about 20%, and increase in specific activity based on protein concentration was about 300 times at this stage.

Dose response of TSTGF activity by partially purified TSTGF

To study dose response curve and the titer of TSTGF, a part of the above-mentioned partially purified TSTGF was concentrated about 100-fold by ultrafiltration with the use of Amicon 8400 cell and YM5 membrane (Amicon Corp., Lexington, MA). Concentrated and unconcentrated TSTGF were tested for the capacity to support the growth of 9-16 Th cells in comparison with that obtained by recombinant IL2 (rIL2). The dose response curve of TSTGF activity is given in Fig. 7. The results show that TSTGF concentrate reached the maximum response at the dilution of 6.25% and that this maximum response was almost comparable to that obtained by rIL2. The unconcentrated TSTGF sample also showed a similar dose response curve. The titers of the TSTGF activity of concentrated and unconcentrated partially purified TSTGF preparation were calculated by comparison with dose response by TSTGF concentrate. The result demonstrates that the titers were about 60 and 0.7 U/ml respectively in terms of IL-2 titer.

Estimation of molecular weight of TSTGF

To estimate the molecular weight of TSTGF, partially purified TSTGF preparation, after being concentrated 100-fold, was applied to Sephacryl S-200 and the TSTGF activity in each fraction obtained from the chromatography was assessed by its ability to induce the proliferation of 9-16 cells. As shown in Fig. 8, analysis of TSTGF by Sephacryl S-200 gel filtration showed its apparent molecular weight is 25,000. The same concentrated sample was also analyzed by SDS-PAGE (12.5% acrylamide) under nonreducing conditions. Upon completion of the run, the gel was sliced into 0.5 cm pieces and proteins were eluted from each sliced gel. Each fraction was assayed for TSTGF activity with the use of 9-16 Th cells. In each of the 3 separate experiments, TSTGF activity ws reproducibly detected in fractions having a molecular weight of about 25,000. A representative experiment is shown in Fig. 9.

Physicochemical properties of TSTGF

The stability of TSTGF was examined by subjecting partially purified TSTGF preparation to various treatments. The activity was found to be resistant to a moderate heat treatment (65°C, 10 min) but stable under acidic (pH 2.0) and alkaline (pH 9.0) conditions, while the activity partially decreased at pH of around 4.0 -5.0 by some unknown reason (Table 6). In contrast to this, it was susceptible to proteolysis with trypsin and to reduction by DTT (10 mM DDT, 2 hr) (Table 7).

Lack of known cytokine activities in partially purified TSTGF preparation

As mentioned already, no interleukin (IL1, IL2, IL3, IL4, IL5) or interferon activity was not detected in the culture supernatant of TSCF cell line. However, it has already been known that several cytokines induce the proliferation of T cells. These include IL2, IL4 and GM-CSF (J. Immunol. 138:4208, 1987; J. Immunol. 138:4293, 1987). In fact, the results in Table 8 show that 9-16 Th clone used as the target of TSTGF activity is capable of proliferating in the presence of IL2 or IL4 but not of GM-CSF. Therefore, a study was further made as to whether the concentrated partially purified TSTGF contains such known cytokines, especially IL2 or IL4. The results in Table 9 clearly show that the partially purified preparation failed to induce the proliferation of CTLL-2 and HT-2 which can respond to IL2 and/or IL4. No other interleukin or interferon activity was also detected in the purified preparation. It is to be noted here that colony-stimulating factor (CSF) is present in the culture supernatant of TSCF cell line. Therefore, further experiments were made to investigate the nature of CSF contained in the TSCF supernatant and also to examine whether such CSF activity is still contained in or excluded from the partially purified preparation. Since the partially purified TSTGF contained 0.7 U/ml TSTGF activity, TSCF culture supernatant having almost the same titer of TSTGF activity was used as control. The results in Table 10 show that the culture supernatant of TSCF exhibited an appreciable CSF activity in 2 different types of CSF assay. Morphological examination revealed that almost all of the colonies which proliferated in these assays were of macrophage type, indicating that the CSF is to be classified as M-CSF but not as GM-CSF. This was also the case in colonies produced by L cell culture supernatant. Since no TSTGF activity was detected in L cell culture supernatant or WEHI-3 culture supernatant, both having a considerably potent M-CSF activity, it is evident that the TSTGF activity (proliferation of 9-16 Th clone) is not mediated by CSF (M-CSF) contained in TSCF culture supernatant.

What is important in this connection is that such CSF activity (M-CSF, GM-CSF) in TSCF culture supernatant was completely eliminated in the partially purified TSTGF preparation having almost the same titer of TSTGF activity. In summary of the above, these results indicate that TSTGF is different from any of the previously described cytokines including those capable of inducing T cell proliferation such as IL2, IL4 and GM-CSF.

## Example 3

The above study demonstrated that TSTGF functions as a potent T cell growth factor (TCGF) for various antigen-specific helper T cell clones. To evaluate its capacity to induce the growth promotion of T cells on the basis of the physiological significance, the present study has investigated whether the TSTGF contributes to the growth of thymocytes.

## Antibodies and Reagents

Monoclonal antibodies against murine T3 molecule (2C11) (Proc. Natl. Acad. Sci. USA 84: 1374, 1987), murine L3T4 molecule (GK1.5) (J. Immunol. 131: 2445, 1983) and Lyt2 molecule (3.155) (from ATCC) were utilized.

The GK1.5 monoclonal antibody-producing hybridoma cells were inoculated into pristane-primed BALB/c nu/nu mice intraperitoneally and ascitic fluids were collected. Culture supernatants (SNs) from 3.155 and 2C11 monoclonal antibody-producing hybridomas were also collected. Gamma-globulin fractions of cell-free ascites and culture SNs were obtained by precipitation at 40% saturation with ammonium sulfate. Fluorescein isothiocyanate (FITC) was conjugated to GK1.5 or 2C11 monoclonal antibody by regular methods, followed by DE-52 (Whatman Biochemicals Ltd., England) ion-exchange chromatography. The F/P ratio of FITC/GK1.5 or FITC/2C11 was 1.6 or 6.8. FITC-conjugated anti-Lyt2, anti-Thy1.2 and mouse anti-rat IgG were the products of Becton Dickinson Immunocytometry Systems, Mountain View, CA, of Bio Yeda Ltd., Rehovot, Israel and of Jackson Immunoresearch Laboratories, INC., West Grove, PA., respectively.

Ionomycin was obtained from Behring Diagnostics (La Jolla, CA.). Phorbol myristate acetate (PMA) was purchased from Sigma Chemical Co. (St. Louis. MO.).

## Preparation of L3T4⁻ Lyt2⁻ or L3T4⁻ Lyt2⁻ T3⁻ or dull adult thymocytes

The procedure was essentially the same as that previously described (Proc. Natl. Acad. Sci. USA 84: 3856, 1987, J. Immunol. 136: 4075, 1986). Several adult thymuses were dissected and a single cell suspension was prepared. To obtain L3T4⁻ Lyt2⁻ thymocytes, cells were then treated with the rat anti-L3T4 (GK1.5) and rat anti-Lyt2 (3.155) monoclonal antibodies at 4°C followed by treatment with rabbit complement (C) at 37°C for 45 min. The cells were washed and again incubated with the above antibodies at 4°C. The treated cells were washed and then incubated for 1 hr at 37°C on tissue culture grade petri dishes (Corining Glass Works, NY.) pretreated for 2 hr at 37°C with goat anti-mouse Ig (Cappel, Malvern, PA.) that cross-reacts with rat Ig. The nonadherent cells were collected by gently pipetting. This procedure yielded a population of L3T4⁻ Lyt2⁻ thymocytes representing 0.5 - 1.0 % of the starting thymocyte population.

To prepare L3T4⁻ Lyt2⁻ T3⁻ or dull thymocytes, the additional anti-T3 (2C11) monoclonal antibody was incorporated along with anti-L3T4 and anti-Lyt2 antibodies in the complement treatment and panning steps, and goat anti-hamster IgG (Cappel, Malvern, PA.) was added in treatment of plates for panning. Aliquots of prepared thymocytes were used for flow microfluorometric analysis to determine the efficacy of this procedure.

## Immunofluorescence staining and flow microfluorometry (FMF)

The cell preparation and staining procedures were essentially the same as described previously (J. Immunol, 1326: 1178, 1986). Briefly, $1 \times 16^6$ lymphoid cells were incubated at 4°C for 30 min with FITC-conjugated antibodies, washed twice, resuspended and analyzed for fluorescence. These procedures were performed in Hanks' balanced salt solution (without phenol red) containing 0.1% bovine serum albumin (BSA) and 0.1 % sodium azide. FMF analysis was performed by using a FACStar (Becton Dickinson Immunocytometry Systems, CA). All data were collected by using log amplification, and dead cells were rejected from analysis by additional staining with propidium iodide.

## Result

Growth-promoting effect of TSTGF on L3T4⁻ Lyt2⁻ thymocytes by a combined stimulation with PMA

First the whole population of adult thymocytes ($3 \times 10^5$/well) were stimulated in vitro with TSTGF or recombinant interleukins (ILs) in the absence or presence of PMA. The results of Table 12 confirm that rIL4 plus PMA or ionomycin plus PMA can induce strong proliferation of adult thymocytes (Proc. Natl. Acad. Sci. USA 83: 1862, 1986, J. Immunol. 137: 2260, 1986, Proc. Natl. Acad. Sci. USA 84: 3856, 1987, Eur. J. Immunol. 16: 12, 1986), and demonstrate that the growth of unfractionated adult thymocytes is also supported by a combination of TSTGF plus PMA but not by a combination of rIL1 plus PMA or rIL2 plus PMA. These results indicate that TSTGF as well as rIL4 are capable of promoting the growth of some fractions of adult thymocytes in the presence of PMA. It is, however, noted that TSTGF-or IL4-induced growth-supporting effect is only marginally observed when cultures were performed at the responding cell dose of $3 \times 10^4$/well (Exp. 2 of Table 12).

Adult thymocytes are divisible into four populations based on their L3T4 and Lyt2 phenotypes (J. Exp. Med. 162: 802, 1985, J. Immunol. 134: 3632, 1985). One of these populations, the L3T4⁻ Lyt2⁻(double-negative) thymocytes ((5% of total thymocytes), contains within it cells capable of repopulating irradiated thymus glands and generating all the other T cell populations normally found in this organ (J. Exp. Med. 162, 1985). To examine whether this double-negative, immature thymocyte population is supported for its growth by TSTGF plus PMA, we isolated this population from 5 - 6 wk old mice by eliminating L3T4⁺ and/or Lyt2⁺ thymocytes through lytic treatment with anti-L3T4 and -Lyt2 antibodies plus C and subsequent panning on dishes precoated with goat anti-mouse Ig (Cross-reactive with rat Ig). The results of Fig. 10 illustrate that these sequential treatments result in the isolation of the double-negative thymocytes (cell yield, 0.5 -1.0 %; purity, >99%).

The double-negative adult thymocyte population ($3 \times 10^4$/well) was cultured with TSTGF plus PMA. As shown in Table 13, the double-negative thymocytes failed to respond to either stimulant alone, whereas the co-stimulation with these two stimulation resulted in appreciable proliferation of the immature thymoctye population. Fig. 11 also illustrates that the TSTGF promotes the proliferation of double-negative thymocytes in a dose-dependent manner when combined with PMA. In considering that the same cell concentrate ($3 \times 10^4$/well) of unfractionated thymocytes were unable to exhibit potent proliferation in the presence of TSTGF and PMA (Exp. 2 of Table 12), the results of Table 13 indicate that the combination of TSTGF plus PMA induces the growth-promoting effect mainly on the double negative immature adult thymocytes. It is additionally shown in Table 13 that this stimulant-combination is also capable of supporting the growth of fetal thymocytes on 14 day gestation that have been considered to be double-negative (J. Immunol. 133: 1117, 1984, Immunol. Rev. 82: 141, 1984, Immunol. Rev. 82: 79, 1984).

TSTGF induces the growth-promotion of double-negative thymocytes without stimulating IL2- or IL4-dependent autocrine mechanism.

Since the slight or potent proliferation was observed in the entire thymocyte population ($3 \times 10^5$ cells/well) stimulated with PMA plus IL2 or IL4 (Table 12), we have made a comparison of the costimulatory activity for the growth-promotion of double-negative thymocytes between TSTGF and IL2 or IL4. The results of Table 14 demonstrate that IL4, but not IL2, is capable of inducing the costimulatory effect on double-negative cells as efficiently as TSTGF. These observations may raise a possibility that a combination of TSTGF plus PMA induces the production of IL4 by double-negative thymocytes and that the produced IL4 functions to promote the growth of double-negative cells together with PMA. This possibility was investigated by examining the effect of anti-IL4 antibody on the proliferation as induced by TSTGF plus PMA. Thus, double-negative thymocytes were stimulated with a combination of IL2, IL4 or TSTGF plus PMA in the absence or presence of anti-IL4 or anti-IL2 receptor (control) antibody (Fig. 12). The results demonstrate that the inclusion of anti-IL4 but not of anti-IL2 receptor antibody in cultures resulted in appreciable inhibition of the proliferation of double-negative thymocytes stimulated by IL4 plus PMA. In contrast, the proliferation induced by TSTGF plus PMA was not affected by the presence of either type of antibody. These results are compatible with the notion that TSTGF contributes to the growth-promotion of double-negative thymocytes via an IL2- or IL4-independent pathway.

**IL1 has a potent costimulatory activity for the growth of double-negative thymocytes in combination with TSTGF**

The data thus far presented indicate that TSTGF acts on immature thymocytes for their proliferative response but its ability depends on the presence of PMA as a costimulator. Since PMA is a non-physiological chemical, intriguing is the question of whether the function of PMA can be replaced by some of physiological factors. To answer this question, various cytokines of T cell and non-T cell origins were tested for their capability of functioning as a costimulator of TSTGF to support the growth of double-negative thymoctyes. The results shown in Table 15 demonstrate that IL1 can promote the growth of double-negative thymocytes by synergy with TSTGF as efficiently as PMA. The results also shown that IL2 or IL4 has an appreciable co-stimulatory activity for the proliferation of double-negative thymocytes, whereas IL6 failed to synergize with TSTGF for the expansion of double-negative thymocytes (Table 15).

Since IL1 was the most effective co-stimulant, additional experiments were performed to more accurately investigate the synergistic effect between TSTGF and IL1 (Fig. 13). The co-stimulatory effect of IL1 was dose-dependent and low doses of IL1 between 0.02 and 0.2 U/ml still produced appreciable co-stimulatory activity (Fig. 13A). It was also shown that the proliferation of double-negative thymocytes induced by TSTGF plus IL1 exhibited the peak response around 3 days after the initiation of cultures and that the magnitude as well as the timing of the peak response in cultures stimulated with TSTGF plus IL1 coincide with those obtained by TSTGF plus PMA (Fig. 13B).

**Potent proliferation of double-negative thymocytes by a combination of TSTGF, IL1 and IL2 or IL4.**

While IL1 was revealed to be the most effective replacement of PMA as a co-stimulant of TSTGF for the growth of double-negative thymocytes, Table 15 also demonstrated that their proliferation was inducible by another combination of TSTGF plus the principal TCGF (IL2 or IL4) to an appreciable extent. In order to determine whether these two sets of combinations function in an additive/synergistic way, double-negative thymocytes were cultured in the presence of TSTGF, IL1 and IL2 or IL4. The results of Fig. 14 illustrate that addition of 3 cytokines (TSTGF, IL1 and IL2 or TSTGF, IL1 and IL4) to cultures resulted in strikingly enhanced proliferation of double-negative thymocytes when compared to that induced by each combination of the above two cytokines. Thus, it appears that two types of combinations of TSTGF plus IL1 and TSTGF plus IL2 or IL4 can induce an additive growth-promoting effect on the double-negative population.

**Potent proliferation of L3T4⁻Lyt2⁻T3⁻ ᵒʳ ᵈᵘˡˡ adult thymocytes by a mixture of 3 cytokines including TSTGF**

Several recent studies have shown that L3T4⁻Lyt2⁻ thymocytes can be subdivided into discrete subsets and argued that these seem to represent more than just a single linear developmental stream (Nature 326: 79, 1987). Most investigations to date have suggested that early thymocytes (double-negative) whose development in the thymus precedes classical $\alpha$ $\beta$-bearing mature T cells do not express that T cell receptor (TcR) (Nature 326: 79, 1987). Therefore, we finally investigated whether L3T4⁻Lyt2⁻T3⁻ ᵒʳ ᵈᵘˡˡ thymoctyes obtained by depletion of T3ᵇʳⁱᵍʰᵗ double-negative thymocytes can be stimulated by a mixture of cytokines including TSTGF.

To prepare a L3T4⁻Lyt2⁻T3⁻ ᵒʳ ᵈᵘˡˡ thymocyte subset, anti-T3 (2C11) antibody was included along with anti-L3T4 and anti-Lyt2 antibodies in both the complement treatment and panning step. The efficacy of anti-T3 antibody to deplete T3ᵇʳⁱᵍʰᵗ thymocytes is shown in the results of Fig. 15. Double-negative thymocyte population contained both T3⁻ ᵒʳ ᵈᵘˡˡ and T3ᵇʳⁱᵍʰᵗ subsets, whereas T3ᵇʳⁱᵍʰᵗ cell-depleted subset consisted of a large number of T3⁻ and small number of T3ᵈᵘˡˡ thymocytes.

Table 16 investigates the capacity of thus far used cytokines, either alone or in various combinations, to induce the proliferation of L3T4⁻Lyt2⁻T3⁻ᵒʳ ᵈᵘˡˡ thymocyte subpopulation. The results demonstrate that the magnitude of the proliferation was more potent in the stimulation with TSTGF, IL1 and IL2 or IL4 than in any combination of the above two cytokines. Compared to the pattern of the proliferation obtained for double-negative thymocytes (Fig. 14), it should be noted that more than additive growth-promoting effect was observed in the L3T4⁻Lyt2⁻T3⁻ ᵒʳ ᵈᵘˡˡ thymocyte population when stimulated especially with a combination of TSTGF, IL1 and IL4.

17

Table 1

| Capacity of a thymic stroma-derived cell line or its culture SN to support the growth of Th clone in vitro | | | |
|---|---|---|---|
| Expt. | Monolayer[a] or SN | Growth of Th clone[b] : Cell No. (x $10^{-4}$) at | |
| | | Input | Harvest |
| 1 | none | 10.0 | 1.3 |
| | TSCF monolayer | 10.0 | 30.1 |
| | TSCE monolayer | 10.0 | 7.2 |
| | BALB/3T3 monolayer | 10.0 | 5.2 |
| 2 | none | 5.0 | 0.2 |
| | TSCF monolayer | 5.0 | 27.5 |
| | TSCF SN (25%) | 5.0 | 46.2 |

a. Monolayers were prepared from two types (fibroblastic form; TSCF and epithelial form; TSCE) of thymic stroma-derived cell lines or BALB/3T3 cell line.

b. 10 x $10^4$ (Exp. 1) or 5 x $10^4$ (Exp. 2) of 9-16 Th clone was cultured with either monolayers or supernatant (SN) in a 2-ml volume in 24-well culture plates. 9-16 cells were harvested 9 (Expt. 1) or 6 (Expt. 2) days later.

Table 2

| Retention of antigen-reactivity in Th clone after growth-maintenance by TSCF monolayer or its SN | | |
|---|---|---|
| Stimulation[a] with | [$^3$H]TdR uptake by 9-16 (cpm) after pre-culture with[b]: | |
| | TSCF monolayer | TSCF SN |
| none | 199(1.23) | 90(1.12) |
| I-E[k] spleen cells | 1970(1.08) | 222(1.76) |
| KLH + I-E[k] spleen cells | 11290(1.02) | 11570(1.13) |

a. 9-16 cells (1 x $10^4$/well) were stimulated by 2000 R X ray-irradiated C3H/He spleen cells (5 x $10^4$/well) as I-E[k] antigen-presenting cells with or without KLH antigen (8 μg/m ).

b. 9-16 cells were pre-cultured with TSCF monolayer or its SN for 4 weeks in the absence of I-E[k] and KLH antigens. Culture medium was weekly changed with fresh medium for the pre-culture with TSCF mon layer or with fresh medium containing 25% TSCF SN for the pre-culture with TSCF SN.

Table 3

| Lack of IL1, IL2, IL3 or IL4 activity in TSCF culture SN | | | |
|---|---|---|---|
| Assay for | Responding cells | Additions | [³H]TdR uptake |
| IL1[a] | LBRM-33 1A5 → CTLL-2 | none<br>rIL1 2.0 u/ml<br>0.5<br>0.125<br>TSCF SN 25% | 580(1.09)<br>83996(1.06)<br>75671(1.04)<br>40279(1.08)<br>1780(1.96) |
| IL2 | CTLL-2 | none<br>rIL2 5.0 u/ml<br>1.25<br>0.31<br>TSCF SN 25% | 136(1.03)<br>74510(1.00)<br>16068(1.02)<br>1034(1.03)<br>192(1.09) |
| IL3 | FDC-P2 | none<br>WEHI SN[c]25%<br>6.25<br>1.56<br>TSCF SN 25% | 1185(1.15)<br>25269(1.02)<br>20750(1.01)<br>14287(1.02)<br>96(1.02) |
| IL4[b] | Normal B cells | none<br>rIL4 50 u/ml<br>12.5<br>3.15<br>TSCF SN 25% | 2128(1.05)<br>36022(1.01)<br>35187(1.02)<br>24898(1.04)<br>225(1.09) |

a. LBRM-33 1A5 cells were exposed to various dilutions of rIL1 or TSTGF (25% TSCF SN) in the presence of PHA (2 μg/ml). IL1 activity was obtained by measuring IL2 activity produced in these culture SN with the use of IL2-dependent CTLL-2.
b. IL4 activity was assessed by measuring the proliferation of B cells by co-stimulation with anti-mouse IgM and TSCF SN or rIL4 as control.
c. WEHI SN was obtained from culture SN of WEHI-3 cell line.

Table 4

| Effect of TSTGF on the proliferation of various T cell clones | | | | | | |
|---|---|---|---|---|---|---|
| T cell clone[a] | | | [3H]TdR incorporation in the presence of | | | |
| Type | Name | Cell No. | None | IL2 | IL4 | TSTGF |
| Th | 9-16 | $10^4$ | 459 | 64997 | 34119 | 98216 |
| | 8-E | $10^4$ | 646 | 109331 | 148160 | 13572 |
| | 8-5 | $5 \times 10^4$ | 1612 | 103002 | 57001 | 27270 |
| | 2-13 | $5 \times 10^4$ | 91 | 7074 | 23585 | 13564 |
| | BR2 | $5 \times 10^4$ | 87 | 29595 | 3088 | 3017 |
| | GLT2 | $5 \times 10^4$ | 88 | 14665 | 8060 | 313 |
| | HT2 | $10^4$ | 2117 | 198109 | 23135 | 93 |
| | | $5 \times 10^4$ | 525 | 42058 | N.D. | 51 |
| CTL | CT6 | $10^4$ | 70 | 43807 | N.D. | 115 |
| | | $5 \times 10^4$ | 511 | 17107 | 17364 | 889 |
| | CTLL-2 | $10^4$ | 164 | 39032 | 159 | 78 |
| | | $5 \times 10^4$ | 380 | 6580 | 622 | 170 |
| | IMP | $10^4$ | 192 | 24509 | N.D. | 170 |
| | | $5 \times 10^4$ | 64 | 3456 | 232 | 148 |

a. Various T cell clones (1 or 5 x 10⁴/well) were cultured with rIL2 (20 μ./ml), rIL4 (25 μ /ml) or TSTGF (50% v/v) of culture SN condensed into 0.2 volume by a centricon membrane for 2 days in 96-well microplate.

Table 5

| Production of TSTGF by the primary thymic explant culture | | | | |
|---|---|---|---|---|
| Culture SN[a] | | [3H]TdR incorporation of T cell clone[b] | | |
| From: concentration | | Exp.1 (Explant 1) | Exp.2 (Explant 2) | |
| | (%) | 9-16 | 9-16 | CTLL-2 |
| -- | -- | 176(1.07) | 655(1.47) | 134(1.05) |
| TSCF | 50 | N.D. | 41823(1.03) | 119(1.21) |
| monolayer | 25 | 33016(1.05) | 42775(1.00) | 130(1.30) |
| | 12.5 | 19340(1.05) | 36355(1.02) | 149(1.01) |
| Primary | 50 | 6908(1.08) | 14823(1.08) | 174(1.15) |
| thymic | 25 | 6214(1.08) | 6569(1.03) | 394(1.02) |
| explant | 12.5 | 3503(1.08) | 3550(1.02) | 258(1.08) |

a. Culture SN from the primary C3H/HeN thymic explants was obtained 4 weeks (Exp. 1) or 5 weeks (Exp. 2) after the initiation of cultures of thymic cell suspensions.
b. 9-16 Th or CTLL-2 CTL clone (10⁴/well) was cultured in the presence of culture SN at various concentrations indicated.

Table 6

| Stability of TSTGF after heat or pH treatment | | |
|---|---|---|
| Treatment[a] | TSTGF activity[b] | |
| | $^3$H-TdR uptake | % control |
| - | 27784 ± 797 | (100.0) |
| 56°C 30 min | 27308 ± 103 | 98.3 |
| 65°C 10 min | 29912 ± 177 | 107.7 |
| 95°C 3 min | 7696 ± 6 | 27.7 |
| 20°C 2 hr | | |
| pH 2.0 | 20294 ± 682 | 73.0 |
| pH 3.0 | 17171 ± 142 | 61.8 |
| pH 4.0 | 12392 ± 561 | 44.6 |
| pH 5.0 | 15958 ± 620 | 57.4 |
| pH 6.0 | 24599 ± 173 | 88.5 |

a. Partially purified TSTGF preparation was heated (indicated) or added to 3-fold volumes of either 0.2 M glycine-HCl (for pH 2.0), 0.2 M sodium acetate (for pH 5.0) or 0.2 M Tris-HCl (for pH 9.0). Samples were then dialyzed agains HBSS.
b. Dialyzed sample (50 μl) was added to each well of 10$^5$/well 9-16 Th clone. $^3$H-TdR uptake by the same number of 9-16 cells in the absence of TSTGF sample was 120.

Table 7

Effect of trypsin- or DTT-treatment on TSTGF activity

| Group | Treatment[a] (added with) | TSTGF activity | |
|-------|---------------------------|----------------|------------|
| | | $^3$H-TdR uptake | % control |
| A | - | 18548 ± 690 | (100.0) |
| B | Trypsin→ PMSF + BSA | 94 ± 15 | 0.5 |
| C | Trypsin + PMSF + BSA | 29039 ± 5707 | 156.6 |
| D | DDT | 243 ± 43 | 1.3 |

a. Partially purified TSTGF sample was either treated with trypsin (at a final concentration of 15 $\mu$g/ml) at 37°C for 2 hr followed by the addition of phenylmethylsulfonyl fluoride (PMSF) at a final concentration of 1 mM + bovine serum albumin (BSA) (2.5 mg/ml) (Group B), added simultaneously with the same amount of trypsin, PMSF and BSA (Group C) or treated with dithiothreitol (at a final concentration of 50 mM) at 25°C for 2 hr (Group D). After these treatments, samples were dialyzed against HBSS and used for the assay.

Table 8

| Growth-promotion of 9-16 Th clone by IL2 or IL4 but not by GM-CSF | | |
|-------------------------------------------------------------------|----------------|----------------|
| Stimulated with (U/ml) | $^3$H-TdR uptake by 9-16 (cpm) | |
| | $2^0$ | $2^1$ |
| IL2 (1.25) | 41802 ± 4598 | 37376 ± 746 |
| IL4 (50) | 14792 ± 2129 | 11545 ± 1385 |
| GM-CSF[a] (100) | 390 ± 74 | 330 ± 26 |

a. The efficacy of recombinant GM-CSF was confirmed by two different assays. First, 50 U/ml of GM-CSF produced 68.7 ± 4.5 pieces of colonies consisting of granulocytes or macrophages. Second, the proliferation ($^3$H-TdR uptake) of ormal bone marrow cells (5 x 10$^4$) was 19948 in the presence of GM-CSF (50 U/ml) vs 543 in the absence of GM-CSF.

Table 9

| Lack of IL2 and IL4 activity in partially purified TSTGF | | |
|---|---|---|
| Responding cells | Additions[a] | $^3$H-TdR uptake |
| CTLL2 | none | 202 |
| | rIL2 5.0 U/ml | 61725 |
| | 1.25 | 69693 |
| | 0.31 | 43240 |
| | TSTGF 10 U/ml | 177 |
| HT-2 | none | 1241 |
| | rIL4 25 U/ml | 27321 |
| | 12.5 | 30753 |
| | 3.15 | 25022 |
| | TSTGF 10 U/ml | 1012 |

a. CTLL-2 $(1 \times 10^4/$well) or HT-2 $(1 \times 10^4/$well) cells were stimulated with either recombinant IL2 (rIL2), rIL4 or partially purified TSTGF.

Table 10

| Elimination of CSF activity in partially purified TSTGF preparation | | | | | | |
|---|---|---|---|---|---|---|
| Factor[a] | | TSTGF activity[b] | | CSF activity[c] | | |
| | | $^3$H-TdR uptake cpm x $10^3$ | | Total colony No. | | $^3$H-TdR uptake cpm x $10^3$ |
| | | 25% | 12.5% | 10% | 5% | 12.5% | 6.25% |
| Medium | → | 0.4 | - | 0 | - | 1.0±0.2 | - |
| TSCF SN | → | 22.3 | 14.5 | 30.3±1.2 | 19.5±0.5 | 46.9±3.8 | 29.5±3.8 |
| TSTGF | → | 23.9 | 13.1 | 0 | 0 | 2.1±0.3 | 2.0±0.2 |
| L cell SN | → | 0.9 | - | 25.0±2.1 | 17.7±0.3 | 50.3±2.7 | 44.2±5.6 |
| WEHI-3 SN | → | 0.5 | - | 15.3±0.9 | 10.3±0.9 | 45.4±3.1 | 40.9±1.5 |

a. Culture supernatants (SN) from TSCF, L cell or WEHI-3 cell line or partially purified TSTGF preparation was used for TSTGF assay or for CSF assay at the final concentrations indicated.
b. TSTGF activity was assessed by $^3$H-TdR uptake of $10^5$ 9-16 Th clone.
c. Colony formation and $^3$H-TdR incorporation assays were performed with cells from the same bone marrow preparation. Colony formation was determined after 10 day incubation. Incorporation of $^3$H-TdR was measured after 72 hr incuba ion.

23

Table 11

| Comparison of functions and properties between various factors capable of contributing to T cell proliferation | | | | | | |
|---|---|---|---|---|---|---|
| Factor (mice) | Proliferation of: | | | M.W. (KD) | PI | Stability under reducing conditions |
| | CTLL-2 | HT-2 | 9-6 | | | |
| IL2 | + | + | + | 25 | 4.5,5.4 | stable |
| IL4 | - | + | + | 18-21.7 | 6.3 | unstable |
| GM-CSF | - | + .-[a] | - | 22 | 4.8 | stable |
| KTGF | - | + | unknown | 16-23 | 4.8 | unknown |
| TSTGF | - | - | + | 25 | 6.0 | unstable |

a. GM-CSF induced the proliferation of HT-2 cells maintained in some laboratories but failed to proliferate HT-2 cells in our laboratory.

Table 12

Effect of TSTGF plus PMA on the growth of

thymocytes from adult mice

| Stimulation[a] (dose) | $^3$H-TdR uptake[c] (cpm) | | |
| --- | --- | --- | --- |
| | Exp. 1 | Exp. 2 | |
| | $3 \times 10^5$/well | $3 \times 10^5$/well | $3 \times 10^4$/well |
| medium | 1460 ± 292 | 603 ± 60 | 979 ± 108 |
| PMA (10 nM) | 820 ± 10 | 615 ± 17 | 586 ± 137 |
| TSTGF[b] (20 U/ml) | 1737 ± 10 | 3135 ± 134 | 862 ± 62 |
| TSTGF + PMA | 12086 ± 240 | 15934 ± 1950 | 1728 ± 99 |
| rIL1α (2 U/ml) | 842 ± 16 | 662 ± 26 | 499 ± 77 |
| rIL1α + PMA | 1484 ± 30 | 1466 ± 233 | 475 ± 106 |
| rIL2 (2 U/ml) | 690 ± 90 | 997 ± 83 | 431 ± 30 |
| rIL2 + PMA | 3857 ± 248 | 4224 ± 382 | 759 ± 116 |
| rIL4 (30 U/ml) | 2243 ± 44 | 1289 ± 125 | 668 ± 84 |
| rIL4 + PMA | 29643 ± 2960 | 95168 ± 5506 | 3062 ± 274 |
| Ionomycin (0.5 μg/ml) + PMA | 21661 ± 4332 | 44956 ± 5504 | 4652 ± 365 |

a. The whole thymocytes ($3 \times 10^5$ or $3 \times 10^4$/well) from normal 6 wk-old C3H/He mice were cultured with various stimulating agents for 3 days.

b. TSTGF sample was prepared by applying MRL104.8a culture SN on DEAE-Sephacel chromatography and PBE 94 chromatofocusing and by concentrating eluted TSTGF-containing fractions. A unit of TSTGF was defined as the reciprocal of the dilution producing a half-maximal response using 9-16 helper T cell clone.

c. Cells were pulsed with 0.5 μCi/well of $^3$H-TdR for 6 hr and then incorporated radioactivity was determined. Data represent the mean $\pm$ S.E. of triplicate cultures / group.

Table 13

| Synergistic effect of TSTGF plus PMA on the growth of L3T4⁻Lyt2⁻ adult thymocytes or fetal thymocytes on 14 day gestation | | | |
|---|---|---|---|
| Responding cells[a] | Stimulation with | $^3$H-TdR uptake (cpm) | |
| | | Exp. 1 | Exp. 2 |
| L3T4⁻Lyt2⁻ adult thymocytes | medium<br>PMA<br>TSTGF<br>TSTGF + PMA | 500 ± 131<br>285 ± 25<br>1142 ± 142<br>17576 ± 1386 | 651 ± 6<br>733 ± 249<br>740 ± 37<br>10334 ± 206 |
| fetal thymocytes on 14 day gestation | medium<br>PMA<br>TSTGF<br>TSTGF + PMA | 2930 ± 245<br>1357 ± 137<br>2425 ± 376<br>20530 ± 2407 | 3467 ± 251<br>4533 ± 363<br>1700 ± 130<br>12297 ± 1561 |

a. L3T4⁻Lyt2⁻ adult thymocytes ($3 \times 10^4$ well) or fetal thymocytes on 14 day gestation ($2 \times 10^4$ well) were cultured for 3 days with various stimulating agents at the same doses as in Table 12.

Table 14

| Comparison between TSTGF and various ILs on the ability to promote the growth of double negative thymocytes in the presence of PMA | | |
|---|---|---|
| Stimulation with[a] | $^3$H-TdR uptake (cpm) | |
| | PMA(-) | PMA(+) |
| medium | 1082 ± 241 | 1354 ± 189 |
| TSTGF | 3162 ± 184 | 19060 ± 529 |
| rIL2 | 2004 ± 685 | 2250 ± 51 |
| rIL4 | 1950 ± 136 | 17390 ± 576 |
| Ionomycin | 2645 ± 563 | 35381 ± 3283 |

a. Double negative (L3T4⁻Lyt2⁻) adult thymocytes were stimulated with various agents at the same doses in Table 12.

Table 15

| Effect of TSTGF on the growth-promotion of L3T4⁻Lyt2⁻ thymocytes by synergy with various interleukins | | | | |
|---|---|---|---|---|
| Co-stimulating agents[a] | $^3$H-TdR uptake (cpm) | | | |
| | Exp. 1 | | Exp. 2 | |
| | TSTGF(-) | TSTGF(+) | TSTGF(-) | TSTGF(+) |
| medium | 1293 ± 537 | 1657 ± 255 | 2190 ± 1401 | 3895 ± 389 |
| PMA | 603 ± 111 | 12372 ± 1209 | 1424 ± 555 | 19040 ± 2856 |
| rIL1α | 374 ± 21 | 9119 ± 918 | 1224 ± 465 | 21548 ± 24 |
| rIL2 | 628 ± 65 | 5232 ± 854 | 1475 ± 265 | 9873 ± 691 |
| rIL4 | 373 ± 63 | 7045 ± 1327 | 1936 ± 341 | 10007 ± 1001 |
| rIL6[b] | 253 ± 21 | 2398 ± 89 | 2897 ± 637 | 3873 ± 309 |

a. Double negative adult thymocytes (3 x 10⁴/well) were stimulated with various agents (indicated) in the absence or presence of TSTGF (20 U/ml).
b. The concentration of rIL6 was 10 U/ml.

Table 16

| Enhanced proliferation of L3T4⁻Lyt2⁻T3[· or dull] thymocytes by a combination of [TSTGF + IL1] and IL2 or IL4 | | |
|---|---|---|
| Stimulation with[a] | $^3$H-TdR uptake (cpm) | |
| | Exp. 1 | Exp. 2 |
| medium | 609 ± 47 | 692 ± 136 |
| TSTGF | 2278 ± 172 | 1438 ± 327 |
| TSTGF + rIL1α | 9079 ± 267 | 8220 ± 580 |
| TSTGF + rIL2 | 6888 ± 473 | 4654 ± 95 |
| TSTGF + rIL4 | 7836 ± 542 | 6378 ± 1134 |
| TSTGF + rIL1α + rIL2 | 18237 ± 246 | 17109 ± 3857 |
| TSTGF + rIL1α + rIL4 | 26366 ± 1848 | 36641 ± 3563 |
| rIL1α | 937 ± 139 | 482 ± 87 |
| rIL2 | 624 ± 6 | 639 ± 47 |
| rIL4 | 566 ± 123 | 516 ± 237 |
| rIL1α + rIL2 | 6432 ± 584 | 5302 ± 418 |
| rIL1α + rIL4 | 735 ± 342 | 982 ± 102 |

a. L3T4⁻Lyt2⁻T3[· or dull] adult thymocytes (3 x 10$^4$/well) were stimulated with combinations of cytokines indicated for 3 days.

**Claims**

(1) A T cell growth factor which is derived from a thymic stroma.

(2) The T cell growth factor of Claim 1 which is obtained from a culture supernatant of thymic stroma cells.

(3) The T cell growth factor of Claim 1 or 2 which promotes the growth of T cell clones and/or thymocytes.

(4) The T cell growth factor of any one of Claims 1 to 3 wherein said thymic stroma is of a mammalian origin.

(5) The T cell growth factor of Claim 3 wherein said T cell clones are helper T cell clones.

(6) The T cell growth factor of Claim 3 wherein said thymocytes are immature thymocytes (L3T4⁻Lyt2⁻).

(7) The T cell growth factor of any one of Claims 1 to 6 which has the following properties:

(a) Molecular weight; about 25,000;

(b) Isoelectric point; about 6.0;

(c) Resistant to heat treatment: 65° C, 10 min.;

(d) Stable at pH 2.0 - 9.0 ;

(e) Susceptible to trypsin or dithiothreitol ; and

(f) Having disulfide bond(s) in its molecule.

(8) A process for the production of said T cell growth factor of any one of Claims 1 to 7 which comprises culturing thymic stroma cells in a culture medium and collecting a factor promoting the growth of T cells from the supernatant of said culture medium.

(9) The process of Claim 8 wherein said factor is collected by DEAE column chromatography and chromatofocusing.

(10) The process of Claim 8 or 9 wherein said culture is performed for 12 - 14 days.

Fig. 1

Fig. 2

Fig. 3

Fig. 4

Fig. 5

Fig. 6

Fig. 7

Legend:
- o——o : TSTGF unconcentrate (50%)
- •——• : TSTGF concentrate (50%)
- △——△ : rIL2 (2u/ml)

Y-axis: $^3$H–TdR uptake (cpm)

X-axis: Dilution

Fig. 8

Fig. 9

Fig. 10

untreated      L3T4⁻Lyt2⁻ fraction

Control

FITC-αThy1.2

FITC-αL3T4

FITC-αLyt2

FITC-αRat Ig

Relative cell number

Log fluorescence intensity

Fig. 11

EP 0 310 056 A2

Fig. 12

| Stimulation | $^3$H-thymidine uptake (cpm$\times 10^{-3}$) |

EP 0 310 056 A2

Fig. 13

Graph A: ³H-thymidine uptake (cpm×10⁻³) vs Concentration of rIL1α (U/mℓ). Legend: △ rIL1α, ▲ TSTGF + rIL1α, ● TSTGF + PMA.

Graph B: ³H-thymidine uptake vs Days after culture. Legend: □ TSTGF, ○ PMA, △ rIL1α, ● TSTGF + PMA, ▲ TSTGF + rIL1α.

Fig. 14

| Stimulation with | $^3$H-thymidine uptake (cpm$\times 10^{-3}$) | |
|---|---|---|
| | Exp. 1 | Exp. 2 |
| Medium | | |
| TSTGF | | |
| TSTGF + rIL1$\alpha$ | | |
| TSTGF + rIL2 | | |
| TSTGF + rIL4 | | |
| TSTGF + rIL6 | | |
| TSTGF + rIL1$\alpha$ + rIL2 | | |
| TSTGF + rIL1$\alpha$ + rIL4 | | |
| TSTGF + rIL1$\alpha$ + rIL6 | | |
| rIL1$\alpha$ + rIL2 | | |
| rIL1$\alpha$ + rIL4 | | |
| rIL1$\alpha$ + rIL6 | | |

Fig.15